# EUROPEAN PATENT APPLICATION

(11) **EP 4 134 451 A1**
(43) Date of publication of application: **15.02.2023**
(21) Application number: 21190726.6
(22) Date of filing: 11.08.2021
(51) Int. Cl.: C12Q 1/6886

(54) **METHOD FOR IDENTIFYING RESPONSIVENESS TO FIBROBLAST GROWTH FACTOR RECEPTOR 1 INHIBITOR THERAPY**

(71) Applicant: Universität zu Köln, 50923 Köln (DE)
(72) Inventor: MALCHERS, Florian, 50931 Köln (DE); THOMAS, Roman, 50931 Köln (DE)
(74) Representative: Kuttenkeuler, David

(57) **Abstract**

The present invention pertains to a method for identifying a subject suffering from cancer as a responder or non-responder to fibroblast growth factor receptor 1 (FGFRi) inhibitor therapy. The method involves the determination of the presence or absence of a genetic alteration of the *FGFR1* gene, wherein the genetic variation is a copy number variation caused by a Breakage-Fusion-Bridge-like (BFB-like) mechanism wherein the break is located within, or within close proximity of, the ORF of the *FGFR1* gene. These breaks associate with FGFR inhibitor sensitivity, and intra-chromosomal tail-to-tail breaks in *FGFR1* may work as a predictive therapeutic marker for an FGFR inhibitor therapy in cancer that can be used to stratify patients for FGFR-inhibitor therapy. Furthermore provided are inhibitors of FGFR1 for use in the treatment of cancer, and a method for identifying a genetic biomarker associated with a disorder, such as cancer.

## Description

### FIELD OF THE INVENTION

The present invention pertains to a method for identifying a subject suffering from cancer as a responder or non-responder to fibroblast growth factor receptor 1 (FGFR1) inhibitor therapy. The method involves the determination of the presence or absence of a genetic alteration of the *FGFR1* gene, wherein the genetic alteration is a copy number variation caused by chromosomal breaks. Breakage-Fusion-Bridge-like (BFB-like) mechanism is the main cause of these copy number variations and induce breaks within, or within close proximity of, the ORF of the *FGFR1* gene. The breaks lead to deregulated FGFR1 expression or to the deletion of the canonical FGFR1 ATG start-codon, are associated with FGFR inhibitor sensitivity, and intra-chromosomal tail-to-tail breaks in or close to *FGFR1* and may work as a predictive therapeutic marker for an FGFR inhibitor therapy in cancer that can be used to stratify patients for FGFR-inhibitor therapy. Furthermore provided are inhibitors of FGFR1 for use in the treatment of cancer, and a method for identifying a genetic biomarker associated with a disorder, such as cancer.

### DESCRIPTION

The Fibroblast growth factor receptor 1 (FGFR) family of receptor tyrosine kinases comprises five highly conserved members, namely Fibroblast growth factor receptor 1 (FGFR1), Fibroblast growth factor receptor 2 (FGFR2), Fibroblast growth factor receptor 3 (FGFR3), Fibroblast growth factor receptor 4 (FGFR4), and Fibroblast growth factor receptor-like 1 (FGFRLi). A full-length representative protein member of the FGFR family consists of an extracellular region, composed of two or three immunoglobulin-like domains, a single hydrophobic membrane-spanning segment and a cytoplasmic tyrosine kinase domain. The extracellular portion of the protein interacts with fibroblast growth factors, setting in motion a cascade of downstream signals.

FGFR1 is also known as fms-related tyrosine kinase-2 and CD331, and the FGFR1 gene is located on human chromosome 8 at position p11.23 (i.e. 8p11.23). The gene comprises 24 exons, and codes for a precursor mRNA that is alternatively spliced at exons 8A or 8B, thereby generating two mRNAs coding for two different FGFR1 isoforms, FGFRi-IIIb (also termed FGFRib) and FGFRi-IIIc (also termed FGFRic), respectively. While FGFRi-IIIc appears responsible for most of the functions of the FGFR1 gene, FGFRi-IIIb appears to have only a minor, somewhat redundant functional role. Furthermore, the expression of at least nine FGFR1 splice variants are described (Gene: FGFR1 (ENSG00000077782) - Homo_sapiens - Ensembl genome browser 103). Of note, it is likely that during embryonal development, some cells express only the extracellular part of FGFR. Interestingly, the secretion of this part functions as an FGF trap (FGF is the activating ligand of FGFR1) and regulates FGFR-signalling by a FGF-gradient.

Previous studies have disclosed an association between an enhanced expression of FGFR1 and various cancers, such as breast, bladder and lung cancer. Also, somatic mutations and epigenetic changes in the expression of the FGFR1 gene have been found to be linked to various cancers, including glioblastoma, melanoma, breast, hematological and lung cancer. The FGFR1 gene is often activated in human cancers as a result of duplication, fusion with other genes, and point mutations. Accordingly, FGFR1 is classified as proto-oncogene. It is also known that several cancerous diseases are, in principle, dependent on FGFR1. In view of the above, FGFR inhibitors can be a viable therapeutic option in the treatment of cancer patients with gene amplification or increased expression of members of FGFR1. Currently, multiple FGFR inhibitors are in clinical trials.

US20160090633A1 discloses methods for identifying a cancer patient that will be responsive to treatment with a fibroblast growth factor receptor (FGFR) inhibitor and methods of treating cancer patients. The methods involve evaluating a biological sample from the patient for the presence of one or more FGFR mutants from a FGFR mutant gene panel.

EP2695950A1 discloses a method for predicting the responsiveness of a mammalian tumor cell or cancer cell to an FGFR inhibitor comprising the evaluation of the status of FGFR and the status of c-myc, whereby the status of FGFR and of c-myc is indicative for the responsiveness to the inhibitor.

EP3102705A2 discloses a method of characterizing a cancer by obtaining a sample from a subject suspected of having cancer; and determining whether a fibroblast growth factor receptor (FGFR) fusion is present in the sample, wherein the FGFR fusion comprises a FGFR locus, thereby characterizing the cancer based on the presence or absence of the FGFR fusion.

Lung cancer is classified into two diverse types of cancer, namely non-small-cell lung carcinoma (NSCLC) and small-cell lung carcinoma (SCLC). The term NSCLC comprises any type of epithelial lung cancer other than SCLC, and accounts for about 85% of all lung cancers. Unfortunately, most cases of NSCLCs are relatively insensitive to chemotherapy, compared to SCLC. The most common types of NSCLC are squamous cell lung cancer (SQLC), large-cell carcinoma, and adenocarcinoma.

Squamous cell lung cancer (SQLC) is the second most common lung cancer subtype and among the cancers with the worst prognosis. Unfortunately, genetically activated kinase targets that provide opportunities for effective drug treatment, such as those occurring in lung adenocarcinoma (e.g., EGFR mutations or ALK rearrangements) have not been identified so far in squamous cell lung cancer. The discovery of recurrent *FGFR1* amplifications (located on 8p11.23) had raised hopes that patients with such amplifications might benefit from FGFR inhibition. In a clinical trial, only a minority of patients (approx. 10%) of patients with *FGFR1-*amplified tumors exhibited durable responses to single agent FGFR inhibition. Due to the continuing need for novel ways to treat cancers such as NSCLC, in particular SQLC, a reliable method to distinguish fibroblast growth factor receptor-inhibitor therapy responders from non-responders is urgently needed. Thus, the technical problem underlying the present invention is the provision of means and methods for the evaluation of subjects and/or cells for their responsiveness to anti-cancer treatment with inhibitors of the fibroblast growth factor receptor.

The technical problem is solved by provision of the embodiments characterized in the claims.

The present invention solves the above identified technical problem since, as documented herein below and in the appended examples, it was surprisingly found that a Breakage-Fusion-Bridge-like (BFB-like) mechanism can occur within the 8p arm. Interestingly, the BFB-like mechanism induces several head-to-head and tail-to-tail breaks within the 8p arm. Tail-to tail breaks close to the *FGFR1* transcription start site induce focal FGFR1 amplification, deregulate FGFR1 transcription and are associated with FGFR inhibitor sensitivity. Furthermore, tail-to-tail breaks within FGFR1 Exon 1 to Exon 9 induce the deletion of the canonical FGFR1 ATG start codon, and the cancer cell uses a downstream in-frame ATG start codon. This often leads to transcription of a truncated FGFR1 version lagging the extracellular part of FGFR1, which has inhibitory function for FGFR1 signaling. Thus, these tail-to-tail intra-chromosomal breaks in *FGFR1* and close to FGFR1 may work as a predictive therapeutic marker for an FGFR inhibitor therapy in cancer, such as NSCLC, in particular SQLC, that can be used to stratify patients for FGFR-inhibitor therapy. Analysis of the presence or absence of a genetic alteration of the *FGFR1* gene, wherein the genetic alteration is a copy number variation caused by breaks located within, or within close proximity of, the ORF of the *FGFR1* gene provides for the reliable prediction of the responsiveness of a tumor/cancer cell or an individual to cancer/tumor treatment with an FGFR1 inhibitor therapy.

### BRIEF DESCRIPTION OF THE INVENTION

Generally, and by way of brief description, the main aspects of the present invention can be described as follows:
In **a first aspect,** the invention pertains to a method for identifying a subject suffering from cancer as a responder or non-responder to fibroblast growth factor receptor 1 (FGFR1) inhibitor therapy.
In **a second aspect,** the invention pertains to an inhibitor of FGFR1 for use in the treatment of cancer in a patient.
In **a third aspect,** the invention pertains to a method for identifying a subject suffering from cancer as a responder or non-responder to a therapy with a receptor binding agent.
In **a fourth aspect,** the invention pertains to a method for identifying a genetic biomarker associated with a disorder.
In **a fifth aspect,** the invention pertains to a method for the diagnosis, prognosis, stratification and/or monitoring of a therapy, of cancer in a subject.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In the first aspect, the invention pertains to a method for identifying a subject suffering from cancer as a responder or non-responder to fibroblast growth factor receptor 1 (FGFR1) inhibitor therapy, the method comprising the steps of
a) Providing from the subject a biological sample comprising genetic material associated with the cancer,
b) Determining in the genetic material the presence or absence of a genetic alteration of the *FGFR1* gene, wherein the genetic alteration is a copy number variation caused by chromosomal breaks, such as a Breakage-Fusion-Bridge-like (BFB-like) mechanism, wherein the break is located within, or within close proximity of, the ORF of the *FGFR1* gene;
wherein the presence of the genetic alteration in the *FGFR1* gene in the biological sample of the subject indicates that the subject is a responder to FGFRi-inhibitor therapy.

As used herein, the term "subject" or "patient" refers to any animal (e.g., a mammal), including, but not limited to, humans, non-human primates, rodents, and the like, which is to be the recipient of a particular treatment. Typically, the terms "subject" and "patient" are used interchangeably herein in reference to a human subject. In a particularly preferred embodiment, the subject is a mammal, such as a mouse, a rat, a guinea pig, a rabbit, a cat, a dog, a monkey, or a human, preferably a human, such as a human patient, more preferably a human patient at risk of suffering from cancer, and most preferably a human patient having received a lung transplantation, a lung allograft, a graft of hematopoetic stem cells, a chemotherapy, and/or a radiotherapy, optionally wherein said human patient is at risk of suffering from cancer.

As used herein, the term "subject at risk of suffering from cancer" or "human patient at risk of suffering from cancer" refers to a subject that presents one or more symptoms indicative of cancer.

Methods according to the present invention are useful for identifying a subject suffering from cancer as a responder or non-responder to fibroblast growth factor receptor 1 (FGFR1) inhibitor therapy, wherein the cancer is any kind of cancer. In a particularly preferred embodiment, the cancer is a solid cancer, preferably a cancer characterized by the expression of FGFR1, such as breast cancer, a gastric cancer, bladder cancer or lung cancer, and most preferably is Non-small cell lung cancer (NSCLC), such as squamous cell lung cancer (SQLC).

In a preferred embodiment, the method for identifying a subject suffering from cancer as a responder or non-responder to fibroblast growth factor receptor 1 (FGFR1) inhibitor therapy is an *in vitro* or *ex vivo* method. Since the herein described methods are non-invasive, the term "providing a biological" sample shall preferably not be interpreted to include a surgical procedure conducted at the subject. In a preferred embodiment, the subject is a human, preferably a human patient diagnosed with NSCLC, such as SQLC.

A "diagnosis" or the term "diagnostic" in context of the present invention means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

The term "biological sample" as used herein refers to a sample that was obtained and may be assayed for the presence or absence of a genetic alteration of the *FGFR1* gene, wherein the genetic alteration is a copy number variation caused by chromosomal breaks, such as a Breakage-Fusion-Bridge-like (BFB-like) mechanism wherein the break is located within, or within close proximity of, the ORF of the *FGFR1* gene as disclosed with the present invention. The biological sample can include a biological fluid (e.g., blood, cerebrospinal fluid, urine, plasma, serum), tissue biopsy, and the like. Preferably, the biological sample comprises tumour cells of the subject, preferably wherein the biological sample is a tumour biopsy, or a sample of a resected primary tumour.

To understand the underlying mechanism of *FGFR1* dependency, the inventors performed whole genome and transcriptome sequencing of *FGFR1*-amplified primary tumors with unknown response upon FGFR inhibition. In addition, the inventors performed deep sequencing of *FGFR1-*amplified samples whereof the response upon FGFR inhibition was known for some of the samples (see Example section). In both cohorts the inventors identified intra-chromosomal tail-to-tail breaks close to the *FGFR1* transcription start site, being responsible for focal amplification of *FGFR1.* In course of the present invention it was surprisingly found that these specific breaks are preferably caused by a Breakage-Fusion-Bridge-like (BFB-like) mechanism in squamous cell lung cancer. The amplification mechanism induces head-to-head followed by tail-to-tail intra-chromosomal breaks within the 8p arm.

Based on this mechanism the inventors were able to reconstruct the observed 8p11-p12 copy number profile, taking only all genomic breaks within the 8p-arm into account. Further, the inventors were able to show that tail-to-tail breaks close to the transcription start side of *FGFR1* occurred in a multitude of 8p11-p12 amplified samples and led to focal amplification of *FGFR1.* This was primarily observed in cell lines, PDX models and patient samples responding upon an FGFR inhibitor therapy. In addition, the inventors could also show that in some *FGFR1*-amplified samples the break occurred within the open reading frame (ORF) of *FGFR1,* causing expression of a somatically altered variant of *FGFR1* lacking the ectodomain (*ΔEC-FGFR1*)*.* Specifically, the inventors observed the expression of short variants of *FGFR1,* and in particular to an altered FGFR1 transcript that lacks the oncogenic FGFR1 using non-canonical in frame ATG start codons, as shown by IHC staining, transcriptomic sequencing, and functional genomics.

Overexpression of the altered FGFR1 transcript transforms Baf3 cells and lead to an *FGFR1*-dependent phenotype. The inventors' results demonstrate that the truncation of the *FGFR1* ectodomain is a frequent event in 8p11.23-amplified squamous cell lung cancer caused by tail-to-tail breaks. These breaks associate with FGFR inhibitor sensitivity and intra-chromosomal tail-to-tail breaks in *FGFR1* may work as a predictive therapeutic marker for an FGFR inhibitor therapy in squamous cell lung cancer that can be used to stratify patients for FGFR-inhibitor therapy.

Accordingly, the methods and means of the present invention can be used to stratify patients into responders and non-responders to FGFR-inhibitor therapy. The term "stratify" or "stratification" for the purposes of this invention refers to the advantage that the method according to the invention renders possible decisions for the treatment and therapy of the patient, whether it is the hospitalization of the patient, the use, effect and/or dosage of one or more drugs, a therapeutic measure or the monitoring of a course of the disease and the course of therapy or etiology or classification of a disease, e.g., into a new or existing subtype or the differentiation of diseases and the patients thereof.

The methods and means of the present invention can also be used to monitor a therapy of cancer, in particular an FGFR-inhibitor therapy in a cancer patient. The term "monitoring a therapy" means for the purpose of the present invention to observe disease progression in a subject who receives a therapy. In other words, the subject during the therapy is regularly monitored for the effect of the applied therapy, which allows the medical practitioner to estimate at an early stage during the therapy whether the prescribed treatment is effective or not, and therefore to adjust the treatment regime accordingly.

As used herein the term "responsiveness to the inhibitor" or "responsive individual to the inhibitor" means in the context of the present invention that a mammalian tumor cell (or mammalian tumor) or mammalian cancer cell (or mammalian cancer) or an individual suffering from cancer, suspected to suffer from or being prone to suffer from cancer shows a response to an inhibitor of FGFR1 (or treatment with an inhibitor of FGFR1). A person of skill will readily be in the position to confirm that (a) cell(s) or individual(s) predicted to be responsive to an inhibitor of FGFR1 shows indeed a response to the inhibitor of FGFR1 (or to treatment with an inhibitor of FGFR1). For example, a response to an inhibitor may be reflected in a decreased suffering from cancer, such as a diminished and/or halted growth of a tumor and/or a reduction of the size of a tumor, the prevention of the formation of metastases or a reduction of number or size of metastases.

The term "response" as used herein may be reflected in form of a "complete response" or "partial response" of the herein identified individuals/patients or tumor cells. Such a "complete response" or "partial response" can primarily be seen in tumor cells or individuals with genetic modification of the *FGFR1* locus resulting in a truncation of at least a part of an extracellular domain of FGFR1, and/or at targeted genetic copy number gain of a FGFR1 gene, or a modified variant thereof; and/or a consecutive palindromic arrangement of the *FGFR1 gene,* or a partial *FGFR1* gene. Accordingly, a "complete response" or "partial response", in particular a "complete response", is preferred.

In this context, the term "response" can refer to a "tumor shrinkage". "Tumor shrinkage" can refer to a reduced volume of the tumor upon treatment with the FGFR1 inhibitor compared to the initial volume at the start of (i.e. prior to) the treatment with the FGFR1 inhibitor. If the tumor volume is, for example, less than about 65% of the initial volume, the tumor has shrunk upon treatment. A tumor volume of, for example, less than 65% (like of from about 1% to 64%) compared to the initial tumor volume (100%) at the start of (i.e. prior to) the treatment with the FGFR1 inhibitor can represent a "partial response" (or partial remission; e.g. a decrease in the size of a tumor, or in the extent of cancer in the body, in response to treatment with the FGFR1 inhibitor). A "partial response" may encompass a (temporarily) tumor shrinkage/reduction in tumor volume during the course of the treatment compared to the initial tumor volume at the start of the treatment (i.e. prior to treatment).

Preferably, the patient group suffering from cancer and identified as a responder or non-responder to fibroblast growth factor receptor 1 (FGFR1) inhibitor therapy according to the present invention shows a "complete response". The term "complete response" as used herein can refer to the disappearance of all signs of cancer in response to the inhibitor of FGFR1 (or treatment with an inhibitor of FGFR1). The term "complete response" and the term "complete remission" can be used interchangeably herein. For example, a "complete response" may be reflected in the continued shrinkage of the tumor until the tumor has disappeared. A tumor volume of, for example, 0% compared to the initial tumor volume (100%) at the start of (i.e. prior to) the treatment with the FGFR1 inhibitor can represent a "complete response". Thus, the terms "responsiveness to the inhibitor" or "responsive individual to the inhibitor" as used herein may indicate the "complete response" / "complete responsiveness" of the tumor (cells), cancer (cells) or individuals predicted to respond in accordance with the present invention.

Any and all methods and means for identifying a subject suffering from cancer as a responder or non-responder to fibroblast growth factor receptor 1 (FGFR1) inhibitor therapy of this invention can be supplemented by conventional histological analysis of sampled specimens, which would allow an earlier and more specific diagnosis of cancer, making earlier treatment with potentially better outcome possible.

The term "FGFR1 inhibitor" or "inhibitor of FGFR1" or "antagonist of FGFR1" or any similar expressions shall in context of the present invention encompass any compound that has an activity as a modulator of the expression, function and/or stability of FGFR1, or of a variant of FGFR1. In some embodiments, the FGFR1 inhibitor can be selected from a small molecule, a polypeptide, peptide, glycoprotein, a peptide-mimetic, an antigen binding protein (ABP) (for example, an antibody, antibody-like molecule or other antigen binding derivative, or an or antigen binding fragment thereof), a nucleic acid such as a DNA or RNA, for example an antisense or inhibitory DNA or RNA, a ribozyme, an RNA or DNA aptamer, RNAi, siRNA, shRNA and the like, including variants or derivatives thereof such as a peptide nucleic acid (PNA), a genetic construct for targeted gene editing, such as a CRISPR/Cas9 construct and/or a guide nucleic acid (gRNA or gDNA) and/or tracrRNA.

In a preferred embodiment, the FGFR1 inhibitor is a selective or unselective FGFR1 inhibitor, such as an inhibitor of FGFR1, FGFR2, FGFR3, and/or FGFR4, such as an inhibitor selected from the group consisting of NVP-BGJ398, AZD4547, TKI258, JNJ42756493, pemigatinib, lucitanib, Rogaratinib, Debio 1347, FIIN2, LY2874455, PRN1371 and ponatinib.

Another preferred embodiment relates to the method for identifying a subject suffering from cancer as a responder or non-responder to FGFR1 inhibitor therapy, wherein the genetic alteration is
a) a genetic modification of the *FGFR1* locus resulting in a truncation of at least a part of an extracellular domain of FGFR1, and/or
b) a genetic copy number gain of a FGFR1 gene, or a modified variant thereof; and/or
c) a consecutive palindromic arrangement of the *FGFR1 gene,* or a partial *FGFR1 gene*; and/or
d) a genetic modification of the FGFR1 locus resulting in the translation initiation of a non-canonical ATG start-codon.

A variant of FGFR1 is, in some embodiments, a protein comprising an amino acid sequence having at least 60%, 70%, 80%, 90%, preferably at least 80% such as at least 90%, sequence identity to SEQ ID NO: 1 (the human FGFR1 amino acid sequence), and most preferably at least 95% (such as at least 98%) sequence identity to SEQ ID NO: 1. In one preferred embodiment of the invention, the variant of FGFR1 comprises an amino acid sequence with at least 80% sequence identity to the amino acid sequence shown in SEQ ID NO: 1.

As used herein, the terms "identical" or percent "identity", when used anywhere herein in the context of two or more nucleic acid or protein/polypeptide sequences, refer to two or more sequences or subsequences that are the same or have (or have at least) a specified percentage of amino acid residues or nucleotides that are the same (i.e., at, or at least, about 60% identity, preferably at, or at least, 65%, 70%, 75%, 80%, 85%, 90%, 91%, 92%, 93% or 94%, identity, and more preferably at, or at least, about 95%, 96%, 97%, 98%, 99%, or higher identity over a specified region - preferably over their full length sequences - , when compared and aligned for maximum correspondence over the comparison window or designated region) as measured using a sequence comparison algorithms, or by manual alignment and visual inspection (see, e.g., NCBI web site).

In a particularly preferred embodiment, the genetic alteration is an alteration of the *FGFR1* gene resulting in the expression of an ectodomain-deleted variant of FGFR1, preferably wherein the variant of FGFR1 is translated from a non-canonical ATG in exon 1 to exon 9 of *FGFR1.*

A further aspect of the present invention pertains to an inhibitor of FGFR1 for use in the treatment of cancer in a patient, wherein the cancer is characterized by a genome which comprises a genetic alteration in the *FGFR1* gene comprising a consecutive palindromic copy number variation of the *FGFR1* gene, or of a fragment of the *FGFR1* gene. Such a genetic alteration in the *FGFR1* gene comprising a consecutive palindromic copy number variation of the *FGFR1* gene, or of a fragment of the *FGFR1* gene, can be the cause of a deregulated transcription.

A preferred embodiment relates to the inhibitor of FGFR1 for use in the treatment of cancer in a patient, wherein the consecutive palindromic copy number variation is an extraverted palindrome.

A further embodiment pertains to the inhibitor of FGFR1 for use in the treatment of cancer in a patient, wherein the inhibitor of FGFR1 is a small molecular inhibitor, an antigen binding protein, such as an antibody, or any antibody derivative, preferably which specifically binds to and inhibits FGFR, preferably FGFR1.

In a particularly preferred embodiment, the inhibitor of FGFR1 is a selective or unselective inhibitor of FGFR1, FGFR2, FGFR3, and/or FGFR4, preferably of FGFR1, such as an inhibitor selected from the group consisting of NVP-BGJ398, AZD4547, TKI258, JNJ42756493, pemigatinib, lucitanib, Rogaratinib, Debio 1347, FIIN2, LY2874455, PRN1371 and ponatinib.

The inhibitor of FGFR1 for use in the treatment of cancer in a patient according to the present invention is useful for the treatment of any kind of cancer. In a preferred embodiment, the inhibitor of FGFR1 is for use in the treatment of cancer in a patient, wherein the cancer is a solid cancer, preferably a cancer characterized by the expression of FGFR1, such as breast cancer, a gastric cancer, bladder cancer or lung cancer and most preferably is Non-small cell lung cancer (NSCLC), such as squamous cell lung cancer (SQLC).

Yet another preferred embodiment relates to the compound for use in the treatment and/or prevention of a proliferative disease in a subject, wherein the proliferative disease is a cancer which is refractory or resistant to treatment with at least one prior therapy.

In a particularly preferred embodiment, the genetic alteration is a focal amplification of the *FGFR1* gene, or of a fragment of the *FGFR1* gene.

A further embodiment pertains to the inhibitor of FGFR1 for use in the treatment of cancer in a patient, wherein the fragment of the *FGFR1* gene is an expressible sequence and results in a FGFR1 protein variant, preferably an ectodomain-deleted FGFR1 protein variant.

Yet another embodiment relates to the inhibitor of FGFR1 for use in the treatment of cancer in a patient, wherein the treatment comprises a preliminary step of identifying the patient as a responder to an FGFR1 inhibitor therapy, wherein the preliminary step comprises a method according to the first aspect of the present invention.

In another aspect, which can be combined with any of the other aspects and any of the particularly preferred embodiments, the present invention provides a pharmaceutical composition for use in the treatment and/or prevention of cancer as described before, together with a pharmaceutically acceptable carrier and/or excipient.

The pharmaceutical composition of the invention is formulated to be compatible with its intended route of administration. Examples of routes of administration include parenteral, e.g., intrathecal, intra-arterial, intravenous, intradermal, subcutaneous, oral, transdermal (topical), intracerebroventricular, intraparenchymal, intratumoral and transmucosal administration. Generally, a pharmaceutical composition of the invention shall comprise one or more compounds for inhibiting FGFR1, and at least one pharmaceutically acceptable carrier and/or excipient. The pharmaceutical compositions as described herein are particularly useful for use in the herein described methods for treating proliferative diseases.

Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; antibacterial agents such as benzyl alcohol or methyl parabens; antioxidants, such as ascorbic acid or sodium bisulfate; chelating agents such as ethylenediaminetetraacetic acid; buffers such as acetates, citrates or phosphates and agents for the adjustment of tonicity such as sodium chloride or dextrose.

Yet a further aspect of the present invention pertains to the use of an inhibitor of FGFR1 for the manufacture of a medicament for the treatment and/or prevention of a proliferative disease.

The herein disclosed compounds and pharmaceutical compositions are in particular useful in a method of preventing or treating a subject suffering from cancer comprising the administration of a therapeutically effective amount of an inhibitor of FGFR1 to the subject. In yet another aspect of this invention, which can be combined with any one of the other aspects and/or embodiments of this invention, there is provided a method for the treatment and/or prevention of a cancer disease in a subject, the method comprising one or more steps of administering to the subject a therapeutically effective amount of an inhibitor of FGFR1.

Treatment or prevention in some embodiments comprises the administration of a therapeutically effective amount of an FGFR1 inhibitor, wherein said inhibitor is preferably administered to said subject by intravenous, vaginal, oral, intranasal, intrathecal, intra-arterial, intradermal, subcutaneous, transdermal (topical), intracerebroventricular, intraparenchymal, intratumoral, transmucosal, rectal, bronchial, and/or parenteral administration, or by any clinically/medically accepted method.

Additionally preferred is that said therapeutically effective amount of an inhibitor of FGFR1 is provided in form of a tablet, a pill, a capsule, a troche, a dragee, a powder, an aerosol spray, a nasal spray, a suppository, a gel, an ointment, a salve, a cream, and/or a solution.

As used herein, the term "therapeutically effective amount" means that amount of a compound or pharmaceutical composition that will elicit the biological or medical response of a tissue, system, animal or human subject that is being sought, for instance, by a researcher or clinician, in accordance with the herein disclosed invention. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder.

For the prevention or treatment of a disease according to this invention, the appropriate dosage of an inhibitor of FGFR1 (or a pharmaceutical composition comprised thereof) will depend on the type of cancer to be treated, the severity and course of the disease, whether the inhibitor of FGFR1 and/or pharmaceutical composition is administered for preventive or therapeutic purposes, previous therapy, the patient's clinical history, age, size/weight and response to the inhibitor of FGFR1, and the decision of the attending physician. The inhibitor of FGFR1 and/or pharmaceutical composition is suitably administered to the patient at one time or over a series of treatments. If such inhibitor of FGFR1 and/or pharmaceutical composition is administered over a series of treatments, the total number of administrations for a given course of treatment may consist of a total of about 2, 3, 4, 5, 6, 7, 8, 9, 10 or more than about 10 treatments. For example, a treatment may be given once every day (or 2, 3 or 4 times a day) for a week, a month or even several months. In certain embodiments, the course of treatment may continue indefinitely.

Another aspect of the present invention pertains to a method for identifying a subject suffering from cancer as a responder or non-responder to a therapy with a receptor binding agent, the method comprising the steps of
a) Providing from the subject a biological sample comprising genetic material associated with the cancer,
b) Determining in the genetic material the presence or absence of a genetic alteration of a gene encoding for a receptor expressed in a cell associated with the cancer, wherein the genetic alteration is
   i. a copy number gain of the gene encoding for the receptor; and/or
   ii. a variant receptor gene encoding for a variant receptor protein which comprises a truncated extracellular domain, or no extracellular domain, of the receptor;
wherein the presence of the genetic alteration in the biological sample of the subject indicates that the subject is a responder to a therapy with a receptor binding agent.

A preferred embodiment relates to the method for identifying a subject suffering from cancer as a responder or non-responder to a therapy with a receptor binding agent, wherein the receptor binding agent binds specifically to the receptor affected by said genetic alteration, preferably wherein the receptor binding agent is an inhibitor of the receptor.

In another preferred embodiment, the genetic alteration is a copy number gain of the variant receptor gene encoding for the variant receptor protein.

A further aspect of the present invention relates to a method for identifying a genetic biomarker associated with a disorder, the method comprising the steps of:
a) Providing a multiplicity of genetic samples of a patient group known to suffer from the disorder, and a multiplicity of genetic samples of a control group known to not suffer from the disorder;
b) Determining for one or more candidate genetic alterations a significant increased chance of occurrence in the multiplicity of genetic samples of a patient group compared to the multiplicity of genetic samples of a control group, wherein the candidate genetic alteration is the result of a tail-to-tail break from a Breakage-Fusion-Bridge (BFB) mechanism or a normal chromosomal break, optionally wherein a focal amplification of a gene is induced, and preferably is a consecutive palindromic copy number change of a genomic sequence;
wherein if the candidate genetic alteration significantly occurs within the multiplicity of genetic samples of a patient group compared to the multiplicity of genetic samples of a control group, the candidate genetic alteration is a biomarker associated with the disorder.

A "biomarker" or "marker" in the context of the present invention refers to a genetic alteration, which is differentially present in a sample taken from subjects having a certain condition as compared to a comparable sample taken from subjects who do not have said condition.

In one embodiment, the genetic sample is a biological sample comprising genomic DNA, preferably a liquid sample, for example derived from blood, plasma, serum, saliva, urine, or is a tissue sample, such as a sample of a tumor tissue affected by the disorder.

A further embodiment relates to the method for identifying a genetic biomarker associated with a disorder, wherein the disorder is a proliferative disorder, such as a cancer, preferably a liquid or solid cancer. In another preferred embodiment, the cancer is breast cancer, a gastric cancer, bladder cancer or lung cancer, and most preferably is Non-small cell lung cancer (NSCLC), such as squamous cell lung cancer (SQLC).

In a particularly preferred embodiment, the genetic alteration is a copy number gain.

In another embodiment, the genomic sequence comprises a complete or partial sequence of a protein coding gene.

Yet another embodiment pertains to the method for identifying a genetic biomarker associated with a disorder, wherein the partial sequence of the protein coding gene still allows for the expression of a protein from the truncated open reading frame or the palindromic genetic locus, preferably without using the canonical ATG start codon, for example by using a non-canonical ATG in exon 1 to exon 9 of *FGFR1.*

In a further embodiment, the chromosomal break, such as the break of the Breakage-Fusion-Bridge-like (BFB-like) mechanism is located within the Open Reading Frame (ORF) of the protein coding gene, and preferably is in close proximity to the transcriptional start site, wherein a close proximity is within 1 mb, preferably within 800 kb, more preferably within 400 kb from the transcriptional start site.

Yet another embodiment relates to the method for identifying a genetic biomarker associated with a disorder, wherein the partial sequence of the protein coding gene is an expressible sequence and results in a FGFR1 protein variant, preferably an ectodomain-deleted FGFR1 protein variant.

Yet another aspect of this invention, which can be combined with any of the other aspects and any of the particularly preferred embodiments, pertains to a kit comprising means for determining the presence or absence of a genetic alteration of the *FGFR1* gene. Preferably the kit of the invention is a kit for performing a method in accordance with the present invention comprising means for determining the presence or absence of a genetic alteration of the *FGFR1* gene, wherein the genetic alteration is a copy number variation caused by a chromosomal break, such as a Breakage-Fusion-Bridge-like (BFB-like) mechanism, wherein the break is located within, or within close proximity of, the ORF of the *FGFR1* gene.

Optionally, the kit can further comprise instructions for suitable operational parameters in the form of a label or a separate insert, and/or a container for holding its ingredients. For example, the kit may have standard instructions informing a consumer how to wash the probe after a sample of plasma is contacted on the probe.

In a further aspect of this invention, which can be combined with any of the other aspects and any of the particularly preferred embodiments, the present invention provides a kit comprising:
i) a therapeutically effective amount of a compound for use according to this invention, or a pharmaceutical composition for use according to this invention,
ii) written instructions to apply said therapeutically effective amount of said compound for use, or said pharmaceutical composition for use; and
iii) optionally, a container holding said compound, or said pharmaceutical composition, and the written instructions.

Optionally, the kit can further comprise instructions for suitable operational parameters in the form of a label or a separate insert. For example, the kit may have standard instructions informing a consumer how to wash the probe after a sample of plasma is contacted on the probe. In either embodiment, the kit may optionally further comprise a standard or control information so that the test sample can be compared with the control information standard.

Yet a further aspect of the present invention pertains to a method for the diagnosis, prognosis, stratification and/or monitoring of a therapy, of cancer in a subject, comprising the steps of:
a) Providing a biological sample from the subject, and
b) Determining in the biological sample the presence or absence of a chromosomal break, such as a Breakage-Fusion-Bridge-like (BFB-like) mechanism, wherein the break is located within, or within close proximity of, the ORF of the *FGFR1* gene, an alteration of the mRNA-transcript of the *FGFR1* gene, an alteration of the FGFR1 protein, an altered level of an mRNA-transcript of the *FGFR1* gene, and/or an altered level of the FGFR1 protein, preferably wherein the extracellular domain of FGFR1 is deleted,
wherein the presence of the chromosomal break, the alteration of the mRNA-transcript of the *FGFR1* gene, the alteration of the FGFR1 protein, a differential level of an mRNA-transcript of the *FGFR1* gene, and/or a differential level of the FGFR1 protein as determined in step (b) compared to a healthy control or reference value is indicative for the presence of cancer in the subject.

In a preferred embodiment of the above diagnostic method, the chromosomal break is a tail-to-tail Breakage-Fusion-Bridge-like (BFB-like) mechanism, wherein the break is located within, or within close proximity of, the ORF of the *FGFR1* gene.

In another preferred embodiment, the chromosomal break induces:
a. a genetic modification of the *FGFR1* locus resulting in a truncation of at least a part of an extracellular domain of FGFR1, and/or
b. a focal amplification of *FGFR1,* or of a fragment of the *FGFR1* gene; and/or
c. a consecutive palindromic arrangement of the *FGFR1 gene,* or a partial *FGFR1* gene; and/or
d. a genetic modification of the FGFR1 locus resulting in the translation initiation of a non-canonical ATG start-codon.

Further preferred is that the presence of the genetic modification of the *FGFR1* gene, and/or the alteration of the mRNA-transcript of the *FGFR1* gene results in the translation of an N-terminally truncated FGFR1 polypeptide. The method therefore preferably comprises detecting N-terminally truncated FGFR1 in a biological sample from the subject. More preferably, the method comprises the detection of a truncated FGFR1 polypeptide, wherein at least one IG-like domain is missing. In a particularly preferred embodiment, the determining of step b) of the method for the diagnosis, prognosis, stratification and/or monitoring of a therapy, of cancer in a subject is performed by immunohistochemistry (IHC).

In yet another preferred embodiment, the truncated FGFR1 variant is detected in step b) of the method for the diagnosis, prognosis, stratification and/or monitoring of a therapy, of cancer in a subject by the RNA technology "Archer" (Democratizing precision oncology (archerdx.com) or (Archer FusionPlex Comprehensive Thyroid & Lung (CTL) | Diagnóstica Longwood (dlongwood.com)).

In yet another preferred embodiment, the genetic alteration, the alteration of the mRNA-transcript of the *FGFR1* gene, or the alteration of the FGFR1 protein comprises a sequence according to any one of SEQ ID Nos: 2 to 37.

Another preferred embodiment relates to the above method for the diagnosis, prognosis, stratification and/or monitoring of a therapy, of cancer in a subject, wherein the genetic alteration, and/or the alteration of the mRNA-transcript of the *FGFR1* gene comprises a nucleic acid sequence according to any one of SEQ ID Nos: 2, 3, 10, 11, 15, 16, 20, 21, 25, 28, 31, 34, or 37, and/or wherein the altered FGFR1 protein comprises an amino acid sequence according to any one of SEQ ID Nos: 9, 14, 19, 24, 26, 29, 32, or 35.

The above method for the diagnosis, prognosis, stratification and/or monitoring of a therapy, of cancer in a subject is useful for diagnosing, prognosing, stratifying and/or monitoring any kind of cancer. Preferably, the cancer is a solid cancer, more preferably a cancer characterized by the expression of FGFR1, such as breast cancer, a gastric cancer, bladder cancer or lung cancer, and most preferably is Non-small cell lung cancer (NSCLC), such as squamous cell lung cancer (SQLC).

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

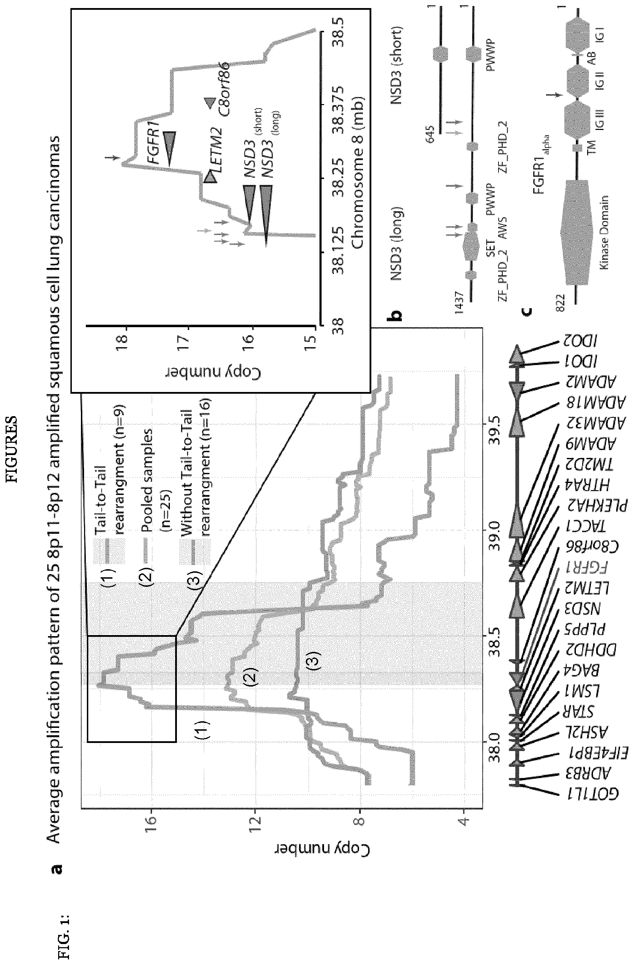

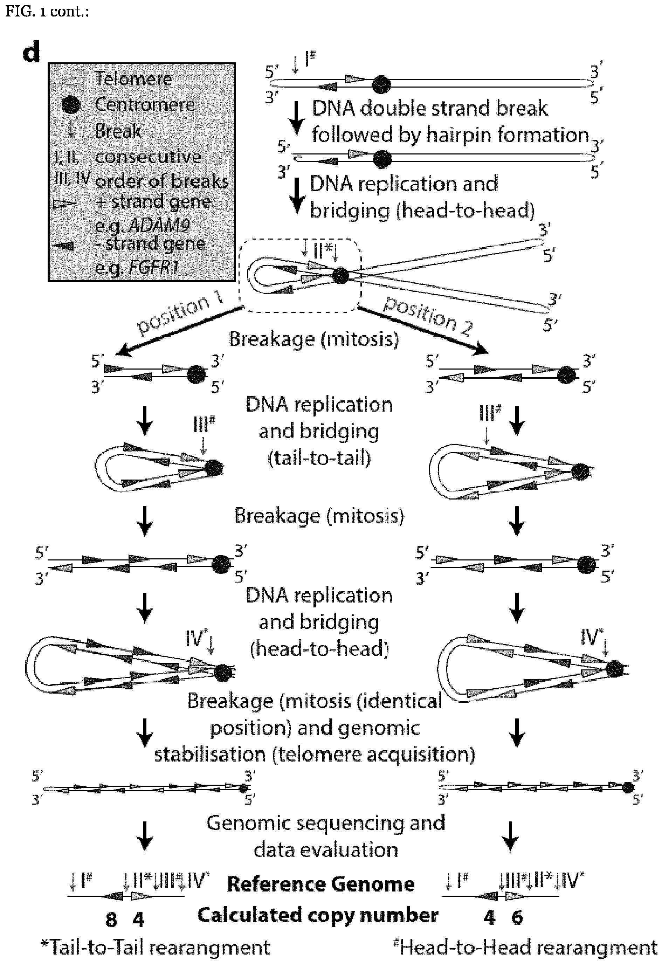

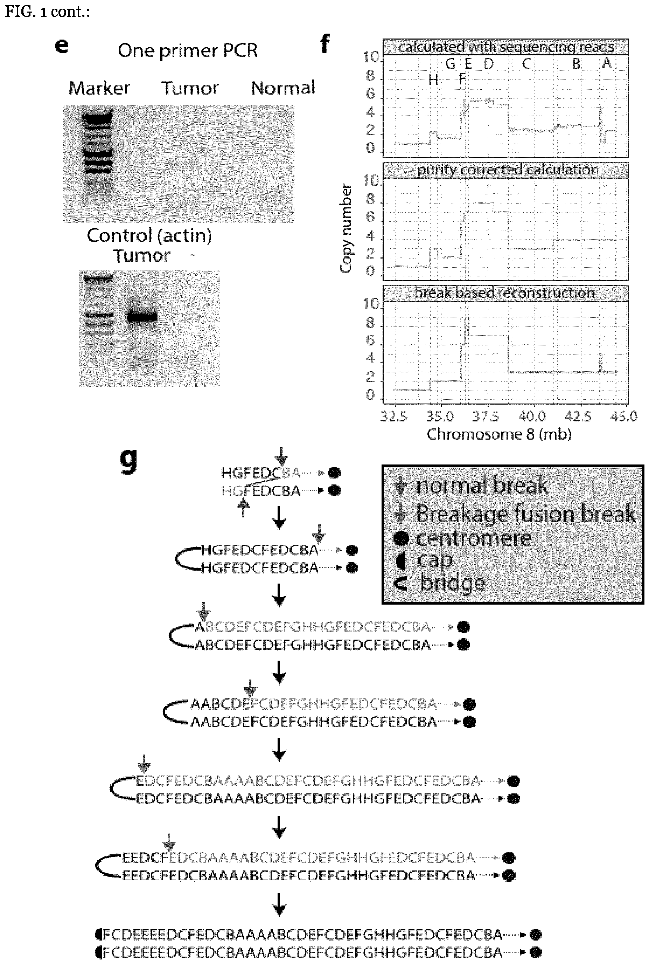

**Figure 1** shows copy number analysis of samples with unknown FGFR inhibitor response. **a)** Average copy number plotted for 25 primary squamous cell lung cancer tumors with 8p11-p12 amplification at chromosome 8p11-p12. Copy numbers were calculated from tumor/normal whole-genome sequencing data and plotted with ROBOCOP (40x average sequencing depth). Line number 2 indicates average copy number of 25 samples with unknown FGFR inhibitor response. Line number 1 indicates average copy number of 9 primary tumor samples with tail-to-tail breaks close to *FGFR1* (400kb range). Line number 3 indicates average copy number of 16 primary tumor samples without tail-to-tail breaks close to *FGFR1* (>1 mb). Locations of genes are annotated with a right-pointing triangle (coded on positive strand) or a left-pointing triangle (coded on negative strand). *FGFR1* location is highlighted, and location of tail-to-tail breaks close to *FGFR1* is also highlighted (left graph). Amplification peak of 9 samples is zoomed (right graph). Location of *FGFR1* and *NSD3* together with detected head-to-head (medium grey arrows), normal (light grey arrow), and tail-to-tail (black arrow) breaks within the genes are indicated. **b)** Domain architectures of NSD3 short (top, 1-645 AA) and NSD3 long (bottom, 1-1437 AA). Medium grey arrows indicate head-to-head, light grey arrows indicate normal breaks. N-terminus is right, C-terminus is left. **c)** Domain architecture of FGFR1 alpha (three IG domains). Detected tail-to-tail break is indicated (black arrow). TM= transmembrane domain, AB = acid box (self-inhibitory function), N-terminus (right) to C-terminus (left, 1-822 AA) **d)** Schematic overview of two random occurring breakage-fusion-bridge mechanisms forming 8p11-p12 amplifications. The two examples differ only in the consecutive order of breaks (indicated by arrows and I, II, III, and IV) **e)** One primer nested PCR over a detected head-to-head break (palindromic) using tumor-, matching normal-DNA (top) and the corresponding control (bottom, actin primers and no template) run at once and on the same gel **f)** Copy number plots of one tumor/normal whole genome sequenced sample (60x sequencing depth). Estimated copy number plot calculated from read counts (top), integral copy number plot calculated from read counts corrected by purity estimation (middle), and a copy number plot generated only from detected breaks and their coverage taking a breakage-fusion-bridge (BFB) mechanism into account (bottom). **g)** Evolution of the BFB shown in Figure 1f (bottom). The 8p arm is indicated by letters starting with A from the centrosome. In total 7 breaks were detected. One normal break (indicated by medium grey arrow) and 6 Breakage-Fusion-Bridge breaks (indicated by the first bridge and black arrows).

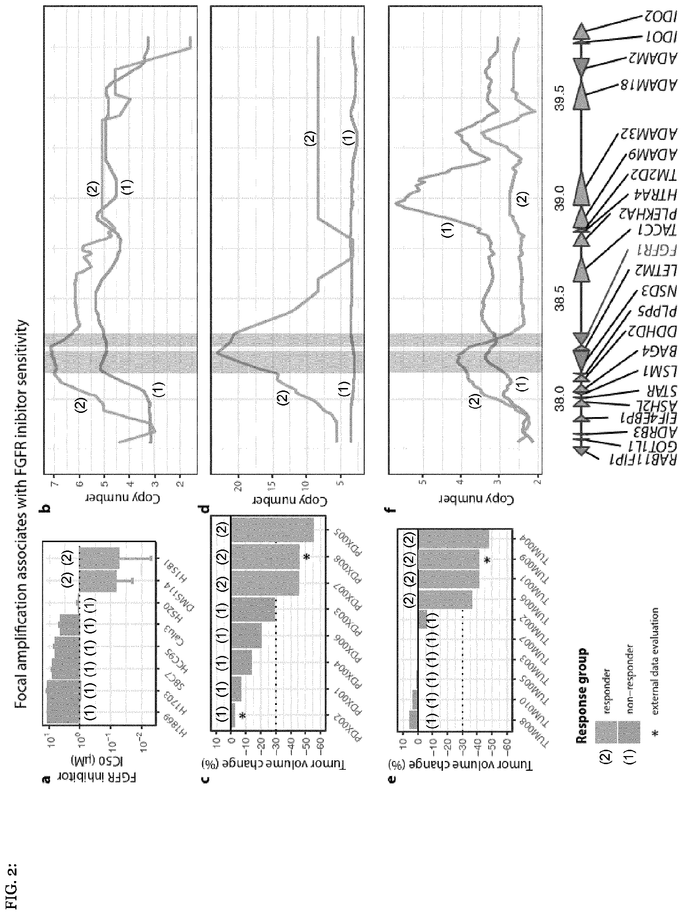

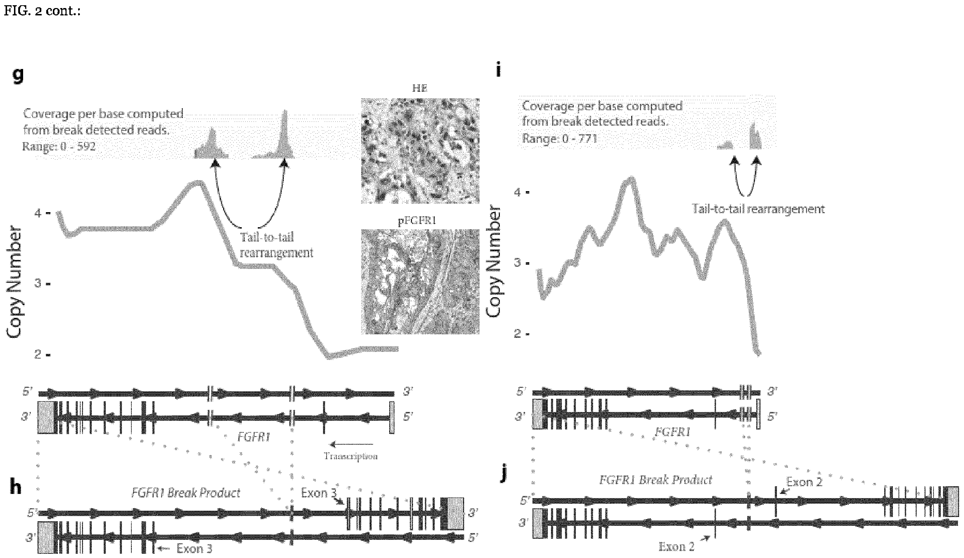

**Figure 2** shows copy number analysis of samples with known FGFR inhibitor response. **a)** Average GI50 values of 8 cell lines screened against the FGFR-inhibitors BGJ398 and AZD4547. Bars are indicated by number (1) (GI50 > 1 µM) or number (2) (GI50 > 1 µM) **b)** Average copy number of 6 cell lines not sensitive upon FGFR inhibition (marked by number (1)) and 2 cell lines which are sensitive upon FGFR inhibition (marked by number (2)). Copy number was extracted from genomic sequencing or whole exome sequencing data and plotted to chromosome 8 (hg19) with ROBOCOP. Location of *NSD3* and *FGFR1* with grey-backed surface **c)** Average tumor growth reduction of 8 patient derived xenograft tumor models treated with BGJ398 vs. vehicle. All models were confirmed to be *FGFR1* amplified by *FGFR1* FISH. Each bar represents an experiment of at least six mice and 12 tumors. Bars are marked by number (1) (tumor reduction of < 30 %) or by number (2) (tumor reduction of > 30 %). **d)** Average copy number of 5 patient derived xenograft tumor models not benefitting from FGFR inhibition (marked by number (1)) and 3 patient derived xenograft tumor (PDX) models which benefit from FGFR inhibition (marked by number (2)). Copy number was extracted from genomic sequencing data and plotted to chromosome 8 (hg19) with ROBOCOP. **e)** Tumor volume change of patients with squamous cell lung cancer harboring FGFR1 amplification and treated with BGJ398. Tumor volume change was measured by RECIST criteria. 5 patients had a progressive disease upon FGFR inhibition (marked by number (1)) and 3 patients showed durable, partial response upon FGFR inhibition (marked by number (2)). TUM003 and TUM007 died during treatment and no sign of response. * labeled sample was an of label FGFR inhibitor treated patient where no RECIST data were available. **f)** Average copy number of 5 patients with progressive disease (marked by number (2)) and 4 patients with durable, partial response upon FGFR inhibition (marked by number (1), one patient was treated with an off label used FGFR inhibitor (GW786034 HCl). Copy number was extracted from hybrid capture based sequencing and plotted to chromosome 8 (hg19) with ROBOCOP. **g)** Hybrid-capture-based genomic sequencing (558x depth) copy number plot zoomed to the location of *FGFR1* (middle). Tumor sample from a patient responding upon treatment with an off label used FGFR inhibitor (GW786034 HCl). Normal exon structure of *FGFR1* with genomic breaks are shown (bottom). Break coverage (top) and location of breaks are indicated (arrows). Corresponding HE and pFGFRi IHC stains are shown (right) **h)** Resulting rearrangement of the tail-to-tail break is shown (dashed lines). **i)** Hybrid-capture-based genomic sequencing (615x depth) copy number plot zoomed to the location of *FGFR1* (middle). Tumor sample is from a patient responding upon treatment with an FGFR inhibitor (BGJ398). Normal exon structure of *FGFR1* (bottom), location of a tail-to-tail break (arrows), and break coverage (top) is shown. **j)** Resulting rearrangement of the tail-to-tail break is shown (dashed lines).

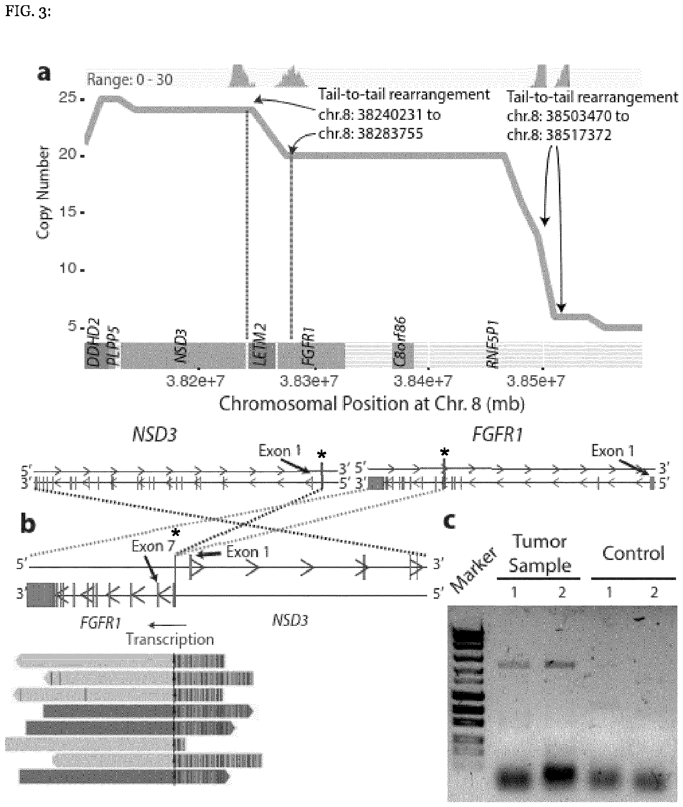

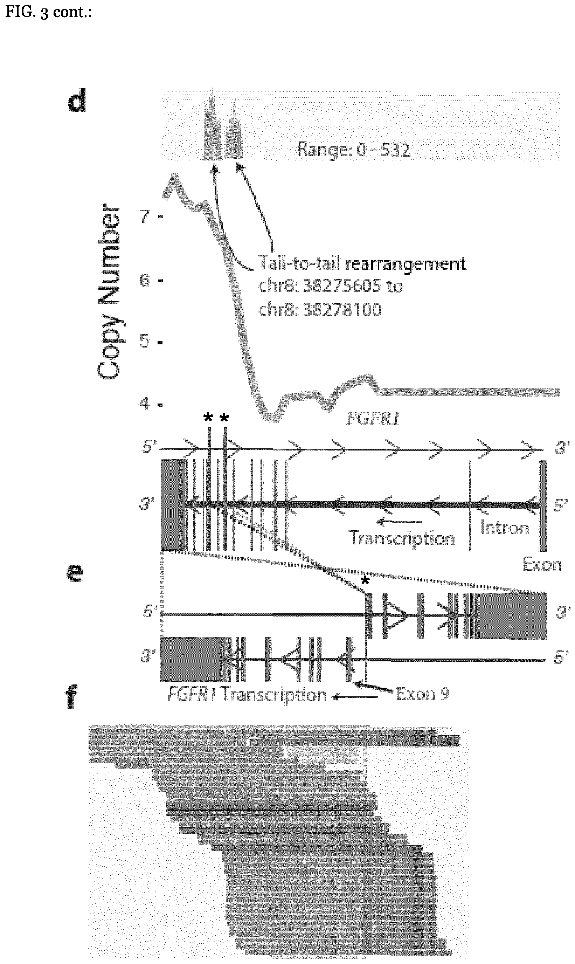

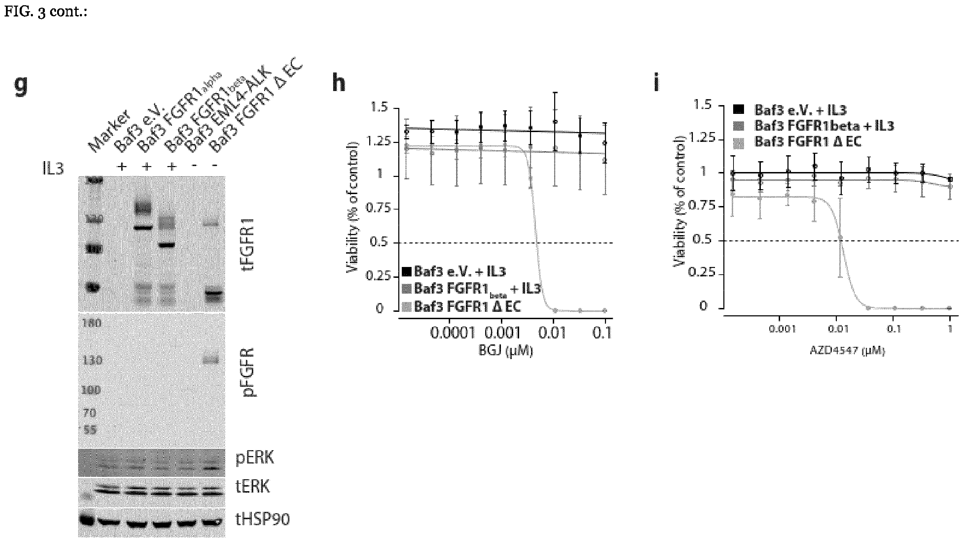

**Figure 3** shows the identification of a novel oncogenic ectodomain deleted FGFR1 version. **a)** Copy number plot of a tumor/normal whole-genome-sequenced (30x coverage) primary squamous cell lung cancer tumor and plotted with ROBOCOP (middle). Located genes in this region (middle, bottom; positive strand, negative strand), break coverage (top), location of tail-to-tail breaks (arrows and black dashed lines) are shown. Normal exon structure of *NSD3* and *FGFR1* with the genomic breaks are indicated by an asterisk independent of the chromosomal position shown above (bottom). **b)** Resulting rearrangement of the t-to-t break (dashed lines, top) and spanning reads from whole transcriptome sequencing data (bottom) is shown. **c)** PCR from corresponding cDNA with two different primer pairs, spanning the t-to-t break and *FGFR1* (left) and matched normal cDNA (right) **d)** Hybrid-capture-based genomic sequencing (468x depth) copy number plot zoomed to the location of *FGFR1* (middle). Tumor sample is from a patient with unknown response upon FGFR inhibition. Normal exon structure of *FGFR1* (bottom), location of a tail-to-tail break (arrows), and break coverage (top) is shown. **e)** The resulting rearrangement of the t-to-t break is shown (dashed lines). **f)** Break spanning reads detected by ARCHER sequencing technique are indicated. **g)** Immunoblotting of transformed Baf3 cells (Baf3 FGFR1 ΔEC and Baf3 EML4-ALK as control) vs. non-transformed Baf3 cells transduced with empty Vector (Baf3 e.V.), FGFR1 alpha (Baf3 FGFR1ₐₚₗₚₐ), and FGFR1 beta (Baf3 FGFR1_{beta}). Baf3 e.V, FGFR1 alpha and beta are cultured with IL3. **h)** Screening of Baf3 e.V., FGFR1 beta, and ectodomain lacking *FGFR1* (FGFR1 ΔEC, using an inframe ATG in exon 9, NM_015850) using increasing concentrations of the FGFR inhibitor BGJ398 or **i)** the FGFR inhibitor AZD4547 (measuring cellular ATP content after 96 hours). Baf3 e.V. and Baf3 FGFR1 beta was screened with IL3 and Baf3 FGFR1 ΔEC was screened under IL3 depleted conditions.

The sequences show:
**SEQ ID NO. 1: Amino acid sequence of human Fibroblast growth factor receptor 1 (FGFR1)**

Met Trp Ser Trp Lys Cys Leu Leu Phe Trp Ala Val Leu Val Thr Ala Thr Leu Cys Thr Ala Arg Pro
Ser Pro Thr Leu Pro Glu Gln Ala Gln Pro Trp Gly Ala Pro Val Glu Val Glu Ser Phe Leu Val His Pro
Gly Asp Leu Leu Gln Leu Arg Cys Arg Leu Arg Asp Asp Val Gln Ser Ile Asn Trp Leu Arg Asp Gly
Val Gln Leu Ala Glu Ser Asn Arg Thr Arg Ile Thr Gly Glu Glu Val Glu Val Gln Asp Ser Val Pro Ala
Asp Ser Gly Leu Tyr Ala Cys Val Thr Ser Ser Pro Ser Gly Ser Asp Thr Thr Tyr Phe Ser Val Asn Val
Ser Asp Ala Leu Pro Ser Ser Glu Asp Asp Asp Asp Asp Asp Ser Ser Ser Glu Glu Lys Glu Thr
Asp Asn Thr Lys Pro Asn Arg Met Pro Val Ala Pro Tyr Trp Thr Ser Pro Glu Lys Met Glu Lys Lys
Leu His Ala Val Pro Ala Ala Lys Thr Val Lys Phe Lys Cys Pro Ser Ser Gly Thr Pro Asn Pro Thr Leu
Arg Trp Leu Lys Asn Gly Lys Glu Phe Lys Pro Asp His Arg Ile Gly Gly Tyr Lys Val Arg Tyr Ala Thr
Trp Ser Ile Ile Met Asp Ser Val Val Pro Ser Asp Lys Gly Asn Tyr Thr Cys Ile Val Glu Asn Glu Tyr
Gly Ser Ile Asn His Thr Tyr Gln Leu Asp Val Val Glu Arg Ser Pro His Arg Pro Ile Leu Gln Ala Gly
Leu Pro Ala Asn Lys Thr Val Ala Leu Gly Ser Asn Val Glu Phe Met Cys Lys Val Tyr Ser Asp Pro
Gln Pro His Ile Gln Trp Leu Lys His Ile Glu Val Asn Gly Ser Lys Ile Gly Pro Asp Asn Leu Pro Tyr
Val Gln Ile Leu Lys Thr Ala Gly Val Asn Thr Thr Asp Lys Glu Met Glu Val Leu His Leu Arg Asn
Val Ser Phe Glu Asp Ala Gly Glu Tyr Thr Cys Leu Ala Gly Asn Ser Ile Gly Leu Ser His His Ser Ala
Trp Leu Thr Val Leu Glu Ala Leu Glu Glu Arg Pro Ala Val Met Thr Ser Pro Leu Tyr Leu Glu Ile Ile
Ile Tyr Cys Thr Gly Ala Phe Leu Ile Ser Cys Met Val Gly Ser Val Ile Val Tyr Lys Met Lys Ser Gly
Thr Lys Lys Ser Asp Phe His Ser Gln Met Ala Val His Lys Leu Ala Lys Ser Ile Pro Leu Arg Arg Gln
Val Thr Val Ser Ala Asp Ser Ser Ala Ser Met Asn Ser Gly Val Leu Leu Val Arg Pro Ser Arg Leu Ser
Ser Ser Gly Thr Pro Met Leu Ala Gly Val Ser Glu Tyr Glu Leu Pro Glu Asp Pro Arg Trp Glu Leu
Pro Arg Asp Arg Leu Val Leu Gly Lys Pro Leu Gly Glu Gly Cys Phe Gly Gln Val Val Leu Ala Glu Ala
Ile Gly Leu Asp Lys Asp Lys Pro Asn Arg Val Thr Lys Val Ala Val Lys Met Leu Lys Ser Asp Ala Thr
Glu Lys Asp Leu Ser Asp Leu Ile Ser Glu Met Glu Met Met Lys Met Ile Gly Lys His Lys Asn Ile Ile
Asn Leu Leu Gly Ala Cys Thr Gln Asp Gly Pro Leu Tyr Val Ile Val Glu Tyr Ala Ser Lys Gly Asn Leu
Arg Glu Tyr Leu Gln Ala Arg Arg Pro Pro Gly Leu Glu Tyr Cys Tyr Asn Pro Ser His Asn Pro Glu
Glu Gln Leu Ser Ser Lys Asp Leu Val Ser Cys Ala Tyr Gln Val Ala Arg Gly Met Glu Tyr Leu Ala Ser
Lys Lys Cys Ile His Arg Asp Leu Ala Ala Arg Asn Val Leu Val Thr Glu Asp Asn Val Met Lys Ile Ala
Asp Phe Gly Leu Ala Arg Asp Ile His His Ile Asp Tyr Tyr Lys Lys Thr Thr Asn Gly Arg Leu Pro Val
Lys Trp Met Ala Pro Glu Ala Leu Phe Asp Arg Ile Tyr Thr His Gln Ser Asp Val Trp Ser Phe Gly Val
Leu Leu Trp Glu Ile Phe Thr Leu Gly Gly Ser Pro Tyr Pro Gly Val Pro Val Glu Glu Leu Phe Lys Leu
Leu Lys Glu Gly His Arg Met Asp Lys Pro Ser Asn Cys Thr Asn Glu Leu Tyr Met Met Met Arg Asp
Cys Trp His Ala Val Pro Ser Gln Arg Pro Thr Phe Lys Gln Leu Val Glu Asp Leu Asp Arg Ile Val Ala
Leu Thr Ser Asn Gln Glu Tyr Leu Asp Leu Ser Met Pro Leu Asp Gln Tyr Ser Pro Ser Phe Pro Asp
Thr Arg Ser Ser Thr Cys Ser Ser Gly Glu Asp Ser Val Phe Ser His Glu Pro Leu Pro Glu Glu Pro Cys
Leu Pro Arg His Pro Ala Gln Leu Ala Asn Gly Gly Leu Lys Arg Arg

According to the present invention, genetic alterations of the *FGFR1* gene are the result of a chromosomal break, such as a tail-to-tail Breakage-Fusion-Bridge (BFB)-like mechanism. These can be detected on several levels, such as on mRNA level. In general, any in-frame non-canonical ATG start codon in exon 1 to exon 9 of *FGFR1* can be used for transcription of altered *FGFR1* after a chromosomal break, such as a Breakage-Fusion-Bridge-like (BFB-like) mechanism. As an example, SEQ ID No: 2 shows the result of a genetic alteration of the *FGFR1* gene that was caused by the tail-to-tail Breakage-Fusion-Bridge (BFB) mechanism.
SEQ ID No: 2

GTGGGAGAGTGACACACAAAACACCCCGGGAAGCCGGAGGAGGCGTGGAAAGGTGGGGCCCG
GGAGGTAAAACTGGGATCGGCGCGTTCAGTGAGGCTGATTAAAGAACAACGCGCAACCAGCG
CCATACGGCGCTCCTCAGGGTGACTCGGCTCTCTAGCTCTTAAGGGTTAGAAGCGACCCTCGC
ACGGTCCCCGGTGCCACCTGGAGCATCATAATGGACTCTGTGGTGCCCTCTGACAAGGGCAAC
TACACCTGCATTGTGGAGAATGAGTACGGCAGCATCAACCACACATACCAGCTGGATGTCGTG
GAGCGGTCCCCTCACCGGCCCATCCTGCAAGCAGGGTTGCCCGCCAACAAAACAGTGGCCCTG
GGTAGCAACGTGGAGTTCATGTGTAAGGTGTACAGTGACCCGCAGCCGCACATCCAGTGGCTA
AAGCACATCGAGGTGAATGGGAGCAAGATTGGCCCAGACAACCTGCCTTATGTCCAGATCTTG
AAGACTGCTGGAGTTAATACCACCGACAAAGAGATGGAGGTGCTTCACTTAAGAAATGTCTCC
TTTGAGGACGCAGGGGAGTATACGTGCTTGGCGGGTAACTCTATCGGACTCTCCCATCACTCT
GCATGGTTGACCGTTCTGGAAGCCCTGGAAGAGAGGCCGGCAGTGATGACCTCGCCCCTGTAC
CTGGAGATCATCATCTATTGCACAGGGGCCTTCCTCATCTCCTGCATGGTGGGGTCGGTCATCG
TCTACAAGATGAAGAGTGGTACCAAGAAGAGTGACTTCCACAGCCAGATGGCTGTGCACAAGC
TGGCCAAGAGCATCCCTCTGCGCAGACAGGTAACAGTGTCTGCTGACTCCAGTGCATCCATGA
ACTCTGGGGTTCTTCTGGTTCGGCCATCACGGCTCTCCTCCAGTGGGACTCCCATGCTAGCAGG
GGTCTCTGAGTATGAGCTTCCCGAAGACCCTCGCTGGGAGCTGCCTCGGGACAGACTGGTCTT
AGGCAAACCCCTGGGAGAGGGCTGCTTTGGGCAGGTGGTGTTGGCAGAGGCTATCGGGCTGG
ACAAGGACAAACCCAACCGTGTGACCAAAGTGGCTGTGAAGATGTTGAAGTCGGACGCAACAG
AGAAAGACTTGTCAGACCTGATCTCAGAAATGGAGATGATGAAGATGATCGGGAAGCATAAGA
ATATCATCAACCTGCTGGGGGCCTGCACGCAGGATGGTCCCTTGTATGTCATCGTGGAGTATG
CCTCCAAGGGCAACCTGCGGGAGTACCTGCAGGCCCGGAGGCCCCCAGGGCTGGAATACTGCT
ACAACCCCAGCCACAACCCAGAGGAGCAGCTCTCCTCCAAGGACCTGGTGTCCTGCGCCTACCA
GGTGGCCCGAGGCATGGAGTATCTGGCCTCCAAGAAGTGCATACACCGAGACCTGGCAGCCAG
GAATGTCCTGGTGACAGAGGACAATGTGATGAAGATAGCAGACTTTGGCCTCGCACGGGACAT
TCACCACATCGACTACTATAAAAAGACAACCAACGGCCGACTGCCTGTGAAGTGGATGGCACC
CGAGGCATTATTTGACCGGATCTACACCCACCAGAGTGATGTGTGGTCTTTCGGGGTGCTCCT
GTGGGAGATCTTCACTCTGGGCGGCTCCCCATACCCCGGTGTGCCTGTGGAGGAACTTTTCAA
GCTGCTGAAGGAGGGTCACCGCATGGACAAGCCCAGTAACTGCACCAACGAGCTGTACATGAT
GATGCGGGACTGCTGGCATGCAGTGCCCTCACAGAGACCCACCTTCAAGCAGCTGGTGGAAGA
CCTGGACCGCATCGTGGCCTTGACCTCCAACCAGGAGTACCTGGACCTGTCCATGCCCCTGGAC

CAGTACTCCCCCAGCTTTCCCGACACCCGGAGCTCTACGTGCTCCTCAGGGGAGGATTCCGTCT
TCTCTCATGAGCCGCTGCCCGAGGAGCCCTGCCTGCCCCGACACCCAGCCCAGCTTGCCAATGG
CGGACTCAAACGCCGCTGACTGCCACCCACACGCCCTCCCCAGACTCCACCGTCAGCTGTAACC
CTCACCCACAGCCCCTGCTGGGCCCACCACCTGTCCGTCCCTGTCCCCTTTCCTGCTGGCAGGA
GCCGGCTGCCTACCAGGGGCCTTCCTGTGTGGCCTGCCTTCACCCCACTCAGCTCACCTCTCCC
TCCACCTCCTCTCCACCTGCTGGTGAGAGGTGCAAAGAGGCAGATCTTTGCTGCCAGCCACTTC
ATCCCCTCCCAGATGTTGGACCAACACCCCTCCCTGCCACCAGGCACTGCCTGGAGGGCAGGG
AGTGGGAGCCAATGAACAGGCATGCAAGTGAGAGCTTCCTGAGCTTTCTCCTGTCGGTTTGGT
CTGTTTTGCCTTCACCCATAAGCCCCTCGCACTCTGGTGGCAGGTGCCTTGTCCTCAGGGCTAC
AGCAGTAGGGAGGTCAGTGCTTCGTGCCTCGATTGAAGGTGACCTCTGCCCCAGATAGGTGGT
GCCAGTGGCTTATTAATTCCGATACTAGTTTGCTTTGCTGACCAAATGCCTGGTACCAGAGGAT
GGTGAGGCGAAGGCCAGGTTGGGGGCAGTGTTGTGGCCCTGGGGCCCAGCCCCAAACTGGGG
GCTCTGTATATAGCTATGAAGAAAACACAAAGTGTATAAATCTGAGTATATATTTACATGTCTT
TTTAAAAGGGTCGTTACCAGAGATTTACCCATCGGGTAAGATGCTCCTGGTGGCTGGGAGGCA
TCAGTTGCTATATATTAAAAACAAAAAAGAAAAAAAAGGAAAATGTTTTTAAAAAGGTCATATA
TTTTTTGCTACTTTTGCTGTTTTATTTTTTTAAATTATGTTCTAAACCTATTTTCAGTTTAGGTCC
CTCAATAAAAATTGCTGCTGCTTCA

The underlined part in SEQ ID No: 2 is being transcribed and is shown as SEQ ID No: 3 below.
SEQ ID No: 3

ATGGACTCTGTGGTGCCCTCTGACAAGGGCAACTACACCTGCATTGTGGAGAATGAGTACGGC
AGCATCAACCACACATACCAGCTGGATGTCGTGGAGCGGTCCCCTCACCGGCCCATCCTGCAA
GCAGGGTTGCCCGCCAACAAAACAGTGGCCCTGGGTAGCAACGTGGAGTTCATGTGTAAGGTG
TACAGTGACCCGCAGCCGCACATCCAGTGGCTAAAGCACATCGAGGTGAATGGGAGCAAGATT
GGCCCAGACAACCTGCCTTATGTCCAGATCTTGAAGACTGCTGGAGTTAATACCACCGACAAA
GAGATGGAGGTGCTTCACTTAAGAAATGTCTCCTTTGAGGACGCAGGGGAGTATACGTGCTTG
GCGGGTAACTCTATCGGACTCTCCCATCACTCTGCATGGTTGACCGTTCTGGAAGCCCTGGAAG
AGAGGCCGGCAGTGATGACCTCGCCCCTGTACCTGGAGATCATCATCTATTGCACAGGGGCCT
TCCTCATCTCCTGCATGGTGGGGTCGGTCATCGTCTACAAGATGAAGAGTGGTACCAAGAAGA
GTGACTTCCACAGCCAGATGGCTGTGCACAAGCTGGCCAAGAGCATCCCTCTGCGCAGACAGG
TAACAGTGTCTGCTGACTCCAGTGCATCCATGAACTCTGGGGTTCTTCTGGTTCGGCCATCACG
GCTCTCCTCCAGTGGGACTCCCATGCTAGCAGGGGTCTCTGAGTATGAGCTTCCCGAAGACCCT
CGCTGGGAGCTGCCTCGGGACAGACTGGTCTTAGGCAAACCCCTGGGAGAGGGCTGCTTTGG
GCAGGTGGTGTTGGCAGAGGCTATCGGGCTGGACAAGGACAAACCCAACCGTGTGACCAAAG
TGGCTGTGAAGATGTTGAAGTCGGACGCAACAGAGAAAGACTTGTCAGACCTGATCTCAGAAA
TGGAGATGATGAAGATGATCGGGAAGCATAAGAATATCATCAACCTGCTGGGGGCCTGCACGC

AGGATGGTCCCTTGTATGTCATCGTGGAGTATGCCTCCAAGGGCAACCTGCGGGAGTACCTGC
AGGCCCGGAGGCCCCCAGGGCTGGAATACTGCTACAACCCCAGCCACAACCCAGAGGAGCAGC
TCTCCTCCAAGGACCTGGTGTCCTGCGCCTACCAGGTGGCCCGAGGCATGGAGTATCTGGCCT
CCAAGAAGTGCATACACCGAGACCTGGCAGCCAGGAATGTCCTGGTGACAGAGGACAATGTGA
TGAAGATAGCAGACTTTGGCCTCGCACGGGACATTCACCACATCGACTACTATAAAAAGACAAC
CAACGGCCGACTGCCTGTGAAGTGGATGGCACCCGAGGCATTATTTGACCGGATCTACACCCA
CCAGAGTGATGTGTGGTCTTTCGGGGTGCTCCTGTGGGAGATCTTCACTCTGGGCGGCTCCCC
ATACCCCGGTGTGCCTGTGGAGGAACTTTTCAAGCTGCTGAAGGAGGGTCACCGCATGGACAA
GCCCAGTAACTGCACCAACGAGCTGTACATGATGATGCGGGACTGCTGGCATGCAGTGCCCTC
ACAGAGACCCACCTTCAAGCAGCTGGTGGAAGACCTGGACCGCATCGTGGCCTTGACCTCCAA
CCAGGAGTACCTGGACCTGTCCATGCCCCTGGACCAGTACTCCCCCAGCTTTCCCGACACCCGG
AGCTCTACGTGCTCCTCAGGGGAGGATTCCGTCTTCTCTCATGAGCCGCTGCCCGAGGAGCCC
TGCCTGCCCCGACACCCAGCCCAGCTTGCCAATGGCGGACTCAAACGCCGCTGA

The primers used for cloning of SEQ ID Nos: 2 and 3 are shown as SEQ ID Nos: 4 to 8 below.
SEQ ID No: 4: CACCTGGAGCATCATAATGGA (212_attB1_S00674)
SEQ ID No: 5: TGGAGCATCATAATGGACTCTG (213_attB1_S00674)
SEQ ID No: 6: AGTCAGCGGCGTTTGAGTC (220_attB2_FGFR1)
SEQ ID No: 7: GTGTGGGTGGCAGTCAGC (221_attB2_FGFR1)
SEQ ID No: 8: CAGTCAGCGGCGTTTGAGT (222_attB2_FGFR1)

Translation of SEQ ID No: 3 generates a protein comprising the amino acid sequence of SEQ ID No: 9.
SEQ ID No: 9

Of note, the altered FGFR1 protein comprising the amino acid sequence of SEQ ID No: 9 has 604 amino acids and due to the N-terminal truncation, the altered FGFR1 protein comprises one IG-like-domain and the protein kinase domain.

FGFR1 transcripts of exemplary primary tumor samples of subjects that are responders to FGFR1-inhibitor therapy are shown in exemplary SEQ ID Nos. below. In principle all variants below can be transcribed (without 5' UTR). As an example, SEQ ID No: 10 shows the transcript of sample A921, where a tail-to-tail-break within the intron before exon 9 was observed.
SEQ ID No: 10

CCCTGGAAGAGAGGCCGGCAGTGATGACCTCGCCCCTGTACCTGGAGATCATCATCTATTGCA
CAGGGGCCTTCCTCATCTCCTGCATGGTGGGGTCGGTCATCGTCTACAAGATGAAGAGTGGTA
CCAAGAAGAGTGACTTCCACAGCCAGATGGCTGTGCACAAGCTGGCCAAGAGCATCCCTCTGC
GCAGACAGGTGTCTGCTGACTCCAGTGCATCCATGAACTCTGGGGTTCTTCTGGTTCGGCCATC
ACGGCTCTCCTCCAGTGGGACTCCCATGCTAGCAGGGGTCTCTGAGTATGAGCTTCCCGAAGA
CCCTCGCTGGGAGCTGCCTCGGGACAGACTGGTCTTAGGCAAACCCCTGGGAGAGGGCTGCTT
TGGGCAGGTGGTGTTGGCAGAGGCTATCGGGCTGGACAAGGACAAACCCAACCGTGTGACCA
AAGTGGCTGTGAAGATGTTGAAGTCGGACGCAACAGAGAAAGACTTGTCAGACCTGATCTCAG
AAATGGAGATGATGAAGATGATCGGGAAGCATAAGAATATCATCAACCTGCTGGGGGCCTGCA
CGCAGGATGGTCCCTTGTATGTCATCGTGGAGTATGCCTCCAAGGGCAACCTGCGGGAGTACC
TGCAGGCCCGGAGGCCCCCAGGGCTGGAATACTGCTACAACCCCAGCCACAACCCAGAGGAGC
AGCTCTCCTCCAAGGACCTGGTGTCCTGCGCCTACCAGGTGGCCCGAGGCATGGAGTATCTGG
CCTCCAAGAAGTGCATACACCGAGACCTGGCAGCCAGGAATGTCCTGGTGACAGAGGACAATG
TGATGAAGATAGCAGACTTTGGCCTCGCACGGGACATTCACCACATCGACTACTATAAAAAGA
CAACCAACGGCCGACTGCCTGTGAAGTGGATGGCACCCGAGGCATTATTTGACCGGATCTACA
CCCACCAGAGTGATGTGTGGTCTTTCGGGGTGCTCCTGTGGGAGATCTTCACTCTGGGCGGCT
CCCCATACCCCGGTGTGCCTGTGGAGGAACTTTTCAAGCTGCTGAAGGAGGGTCACCGCATGG
ACAAGCCCAGTAACTGCACCAACGAGCTGTACATGATGATGCGGGACTGCTGGCATGCAGTGC
CCTCACAGAGACCCACCTTCAAGCAGCTGGTGGAAGACCTGGACCGCATCGTGGCCTTGACCT
CCAACCAGGAGTACCTGGACCTGTCCATGCCCCTGGACCAGTACTCCCCCAGCTTTCCCGACAC
CCGGAGCTCTACGTGCTCCTCAGGGGAGGATTCCGTCTTCTCTCATGAGCCGCTGCCCGAGGA
GCCCTGCCTGCCCCGACACCCAGCCCAGCTTGCCAATGGCGGACTCAAACGCCGCTGACTGCC
ACCCACACGCCCTCCCCAGACTCCACCGTCAGCTGTAACCCTCACCCACAGCCCCTGCTGGGCC
CACCACCTGTCCGTCCCTGTCCCCTTTCCTGCTGGCAGGAGCCGGCTGCCTACCAGGGGCCTTC
CTGTGTGGCCTGCCTTCACCCCACTCAGCTCACCTCTCCCTCCACCTCCTCTCCACCTGCTGGTG

AGAGGTGCAAAGAGGCAGATCTTTGCTGCCAGCCACTTCATCCCCTCCCAGATGTTGGACCAA
CACCCCTCCCTGCCACCAGGCACTGCCTGGAGGGCAGGGAGTGGGAGCCAATGAACAGGCATG
CAAGTGAGAGCTTCCTGAGCTTTCTCCTGTCGGTTTGGTCTGTTTTGCCTTCACCCATAAGCCC
CTCGCACTCTGGTGGCAGGTGCCTTGTCCTCAGGGCTACAGCAGTAGGGAGGTCAGTGCTTCG
TGCCTCGATTGAAGGTGACCTCTGCCCCAGATAGGTGGTGCCAGTGGCTTATTAATTCCGATAC
TAGTTTGCTTTGCTGACCAAATGCCTGGTACCAGAGGATGGTGAGGCGAAGGCCAGGTTGGGG
GCAGTGTTGTGGCCCTGGGGCCCAGCCCCAAACTGGGGGCTCTGTATATAGCTATGAAGAAAA
CACAAAGTGTATAAATCTGAGTATATATTTACATGTCTTTTTAAAAGGGTCGTTACCAGAGATT
TACCCATCGGGTAAGATGCTCCTGGTGGCTGGGAGGCATCAGTTGCTATATATTAAAAACAAA
AAAGAAAAAAAAGGAAAATGTTTTTAAAAAGGTCATATATTTTTTGCTACTTTTGCTGTTTTAT
TTTTTTAAATTATGTTCTAAACCTATTTTCAGTTTAGGTCCCTCAATAAAAATTGCTGCTGCTTC
A

The underlined part in SEQ ID No: 10 is being transcribed and is shown as SEQ ID No: 11 below.
SEQ ID No: 11

ATGACCTCGCCCCTGTACCTGGAGATCATCATCTATTGCACAGGGGCCTTCCTCATCTCCTGCA
TGGTGGGGTCGGTCATCGTCTACAAGATGAAGAGTGGTACCAAGAAGAGTGACTTCCACAGCC
AGATGGCTGTGCACAAGCTGGCCAAGAGCATCCCTCTGCGCAGACAGGTGTCTGCTGACTCCA
GTGCATCCATGAACTCTGGGGTTCTTCTGGTTCGGCCATCACGGCTCTCCTCCAGTGGGACTCC
CATGCTAGCAGGGGTCTCTGAGTATGAGCTTCCCGAAGACCCTCGCTGGGAGCTGCCTCGGGA
CAGACTGGTCTTAGGCAAACCCCTGGGAGAGGGCTGCTTTGGGCAGGTGGTGTTGGCAGAGG
CTATCGGGCTGGACAAGGACAAACCCAACCGTGTGACCAAAGTGGCTGTGAAGATGTTGAAGT
CGGACGCAACAGAGAAAGACTTGTCAGACCTGATCTCAGAAATGGAGATGATGAAGATGATCG
GGAAGCATAAGAATATCATCAACCTGCTGGGGGCCTGCACGCAGGATGGTCCCTTGTATGTCA
TCGTGGAGTATGCCTCCAAGGGCAACCTGCGGGAGTACCTGCAGGCCCGGAGGCCCCCAGGG
CTGGAATACTGCTACAACCCCAGCCACAACCCAGAGGAGCAGCTCTCCTCCAAGGACCTGGTG
TCCTGCGCCTACCAGGTGGCCCGAGGCATGGAGTATCTGGCCTCCAAGAAGTGCATACACCGA
GACCTGGCAGCCAGGAATGTCCTGGTGACAGAGGACAATGTGATGAAGATAGCAGACTTTGGC
CTCGCACGGGACATTCACCACATCGACTACTATAAAAAGACAACCAACGGCCGACTGCCTGTGA
AGTGGATGGCACCCGAGGCATTATTTGACCGGATCTACACCCACCAGAGTGATGTGTGGTCTT
TCGGGGTGCTCCTGTGGGAGATCTTCACTCTGGGCGGCTCCCCATACCCCGGTGTGCCTGTGG
AGGAACTTTTCAAGCTGCTGAAGGAGGGTCACCGCATGGACAAGCCCAGTAACTGCACCAACG
AGCTGTACATGATGATGCGGGACTGCTGGCATGCAGTGCCCTCACAGAGACCCACCTTCAAGC
AGCTGGTGGAAGACCTGGACCGCATCGTGGCCTTGACCTCCAACCAGGAGTACCTGGACCTGT
CCATGCCCCTGGACCAGTACTCCCCCAGCTTTCCCGACACCCGGAGCTCTACGTGCTCCTCAGG

GGAGGATTCCGTCTTCTCTCATGAGCCGCTGCCCGAGGAGCCCTGCCTGCCCCGACACCCAGC
CCAGCTTGCCAATGGCGGACTCAAACGCCGCTGA

The primers used for cloning of SEQ ID Nos: 10 and 11 are shown as SEQ ID Nos: 12 to 13 below.
SEQ ID No: 12 - CCGGCAGTGATGACCTCG (214_attB1_A921)
SEQ ID No: 13 - CAGTGATGACCTCGCCCC (215_attB1_A921)

Translation of SEQ ID No: 11 generates a protein comprising the amino acid sequence of SEQ ID No: 14.
SEQ ID No: 14

Of note, the altered FGFR1 protein comprising the amino acid sequence of SEQ ID No: 14 has 452 amino acids comprising no IG-like-domain due to the N-terminal truncation, but the protein kinase domain. In the context of the present invention, a polypeptide comprising the amino acid sequence of SEQ ID No: 14 is also referred to as ΔEC-FGFR1. The double-underlined residues in SEQ ID No: 14 form the transmembrane domain, whereas the single-underlined residues form the kinase domain.

SEQ ID No: 15 shows the transcript of sample p68.
SEQ ID No: 15

CCGTAGCTCCATATTGGACATCCCCAGAAAAGATGGAAAAGAAATTGCATGCAGTGCCGGCTG
CCAAGACAGTGAAGTTCAAATGCCCTTCCAGTGGGACCCCAAACCCCACACTGCGCTGGTTGA
AAAATGGCAAAGAATTCAAACCTGACCACAGAATTGGAGGCTACAAGGTCCGTTATGCCACCT
GGAGCATCATAATGGACTCTGTGGTGCCCTCTGACAAGGGCAACTACACCTGCATTGTGGAGA
ATGAGTACGGCAGCATCAACCACACATACCAGCTGGATGTCGTGGAGCGGTCCCCTCACCGGC
CCATCCTGCAAGCAGGGTTGCCCGCCAACAAAACAGTGGCCCTGGGTAGCAACGTGGAGTTCA

TGTGTAAGGTGTACAGTGACCCGCAGCCGCACATCCAGTGGCTAAAGCACATCGAGGTGAATG
GGAGCAAGATTGGCCCAGACAACCTGCCTTATGTCCAGATCTTGAAGACTGCTGGAGTTAATA
CCACCGACAAAGAGATGGAGGTGCTTCACTTAAGAAATGTCTCCTTTGAGGACGCAGGGGAGT
ATACGTGCTTGGCGGGTAACTCTATCGGACTCTCCCATCACTCTGCATGGTTGACCGTTCTGGA
AGCCCTGGAAGAGAGGCCGGCAGTGATGACCTCGCCCCTGTACCTGGAGATCATCATCTATTG
CACAGGGGCCTTCCTCATCTCCTGCATGGTGGGGTCGGTCATCGTCTACAAGATGAAGAGTGG
TACCAAGAAGAGTGACTTCCACAGCCAGATGGCTGTGCACAAGCTGGCCAAGAGCATCCCTCT
GCGCAGACAGGTGTCTGCTGACTCCAGTGCATCCATGAACTCTGGGGTTCTTCTGGTTCGGCC
ATCACGGCTCTCCTCCAGTGGGACTCCCATGCTAGCAGGGGTCTCTGAGTATGAGCTTCCCGA
AGACCCTCGCTGGGAGCTGCCTCGGGACAGACTGGTCTTAGGCAAACCCCTGGGAGAGGGCTG
CTTTGGGCAGGTGGTGTTGGCAGAGGCTATCGGGCTGGACAAGGACAAACCCAACCGTGTGA
CCAAAGTGGCTGTGAAGATGTTGAAGTCGGACGCAACAGAGAAAGACTTGTCAGACCTGATCT
CAGAAATGGAGATGATGAAGATGATCGGGAAGCATAAGAATATCATCAACCTGCTGGGGGCCT
GCACGCAGGATGGTCCCTTGTATGTCATCGTGGAGTATGCCTCCAAGGGCAACCTGCGGGAGT
ACCTGCAGGCCCGGAGGCCCCCAGGGCTGGAATACTGCTACAACCCCAGCCACAACCCAGAGG
AGCAGCTCTCCTCCAAGGACCTGGTGTCCTGCGCCTACCAGGTGGCCCGAGGCATGGAGTATC
TGGCCTCCAAGAAGTGCATACACCGAGACCTGGCAGCCAGGAATGTCCTGGTGACAGAGGACA
ATGTGATGAAGATAGCAGACTTTGGCCTCGCACGGGACATTCACCACATCGACTACTATAAAA
AGACAACCAACGGCCGACTGCCTGTGAAGTGGATGGCACCCGAGGCATTATTTGACCGGATCT
ACACCCACCAGAGTGATGTGTGGTCTTTCGGGGTGCTCCTGTGGGAGATCTTCACTCTGGGCG
GCTCCCCATACCCCGGTGTGCCTGTGGAGGAACTTTTCAAGCTGCTGAAGGAGGGTCACCGCA
TGGACAAGCCCAGTAACTGCACCAACGAGCTGTACATGATGATGCGGGACTGCTGGCATGCAG
TGCCCTCACAGAGACCCACCTTCAAGCAGCTGGTGGAAGACCTGGACCGCATCGTGGCCTTGA
CCTCCAACCAGGAGTACCTGGACCTGTCCATGCCCCTGGACCAGTACTCCCCCAGCTTTCCCGA
CACCCGGAGCTCTACGTGCTCCTCAGGGGAGGATTCCGTCTTCTCTCATGAGCCGCTGCCCGA
GGAGCCCTGCCTGCCCCGACACCCAGCCCAGCTTGCCAATGGCGGACTCAAACGCCGCTGACT
GCCACCCACACGCCCTCCCCAGACTCCACCGTCAGCTGTAACCCTCACCCACAGCCCCTGCTGG
GCCCACCACCTGTCCGTCCCTGTCCCCTTTCCTGCTGGCAGGAGCCGGCTGCCTACCAGGGGCC
TTCCTGTGTGGCCTGCCTTCACCCCACTCAGCTCACCTCTCCCTCCACCTCCTCTCCACCTGCTG
GTGAGAGGTGCAAAGAGGCAGATCTTTGCTGCCAGCCACTTCATCCCCTCCCAGATGTTGGAC
CAACACCCCTCCCTGCCACCAGGCACTGCCTGGAGGGCAGGGAGTGGGAGCCAATGAACAGGC
ATGCAAGTGAGAGCTTCCTGAGCTTTCTCCTGTCGGTTTGGTCTGTTTTGCCTTCACCCATAAG
CCCCTCGCACTCTGGTGGCAGGTGCCTTGTCCTCAGGGCTACAGCAGTAGGGAGGTCAGTGCT
TCGTGCCTCGATTGAAGGTGACCTCTGCCCCAGATAGGTGGTGCCAGTGGCTTATTAATTCCG
ATACTAGTTTGCTTTGCTGACCAAATGCCTGGTACCAGAGGATGGTGAGGCGAAGGCCAGGTT
GGGGGCAGTGTTGTGGCCCTGGGGCCCAGCCCCAAACTGGGGGCTCTGTATATAGCTATGAAG
AAAACACAAAGTGTATAAATCTGAGTATATATTTACATGTCTTTTTAAAAGGGTCGTTACCAGA
GATTTACCCATCGGGTAAGATGCTCCTGGTGGCTGGGAGGCATCAGTTGCTATATATTAAAAA

CAAAAAAGAAAAAAAAGGAAAATGTTTTTAAAAAGGTCATATATTTTTTGCTACTTTTGCTGTT
TTATTTTTTTAAATTATGTTCTAAACCTATTTTCAGTTTAGGTCCCTCAATAAAAATTGCTGCTG
CTTCA

The underlined part in SEQ ID No: 15 is being transcribed and is shown as SEQ ID No: 16 below.
SEQ ID No: 16

ATGGAAAAGAAATTGCATGCAGTGCCGGCTGCCAAGACAGTGAAGTTCAAATGCCCTTCCAGT
GGGACCCCAAACCCCACACTGCGCTGGTTGAAAAATGGCAAAGAATTCAAACCTGACCACAGA
ATTGGAGGCTACAAGGTCCGTTATGCCACCTGGAGCATCATAATGGACTCTGTGGTGCCCTCT
GACAAGGGCAACTACACCTGCATTGTGGAGAATGAGTACGGCAGCATCAACCACACATACCAG
CTGGATGTCGTGGAGCGGTCCCCTCACCGGCCCATCCTGCAAGCAGGGTTGCCCGCCAACAAA
ACAGTGGCCCTGGGTAGCAACGTGGAGTTCATGTGTAAGGTGTACAGTGACCCGCAGCCGCAC
ATCCAGTGGCTAAAGCACATCGAGGTGAATGGGAGCAAGATTGGCCCAGACAACCTGCCTTAT
GTCCAGATCTTGAAGACTGCTGGAGTTAATACCACCGACAAAGAGATGGAGGTGCTTCACTTA
AGAAATGTCTCCTTTGAGGACGCAGGGGAGTATACGTGCTTGGCGGGTAACTCTATCGGACTC
TCCCATCACTCTGCATGGTTGACCGTTCTGGAAGCCCTGGAAGAGAGGCCGGCAGTGATGACC
TCGCCCCTGTACCTGGAGATCATCATCTATTGCACAGGGGCCTTCCTCATCTCCTGCATGGTGG
GGTCGGTCATCGTCTACAAGATGAAGAGTGGTACCAAGAAGAGTGACTTCCACAGCCAGATGG
CTGTGCACAAGCTGGCCAAGAGCATCCCTCTGCGCAGACAGGTGTCTGCTGACTCCAGTGCAT
CCATGAACTCTGGGGTTCTTCTGGTTCGGCCATCACGGCTCTCCTCCAGTGGGACTCCCATGCT
AGCAGGGGTCTCTGAGTATGAGCTTCCCGAAGACCCTCGCTGGGAGCTGCCTCGGGACAGACT
GGTCTTAGGCAAACCCCTGGGAGAGGGCTGCTTTGGGCAGGTGGTGTTGGCAGAGGCTATCG
GGCTGGACAAGGACAAACCCAACCGTGTGACCAAAGTGGCTGTGAAGATGTTGAAGTCGGAC
GCAACAGAGAAAGACTTGTCAGACCTGATCTCAGAAATGGAGATGATGAAGATGATCGGGAAG
CATAAGAATATCATCAACCTGCTGGGGGCCTGCACGCAGGATGGTCCCTTGTATGTCATCGTG
GAGTATGCCTCCAAGGGCAACCTGCGGGAGTACCTGCAGGCCCGGAGGCCCCCAGGGCTGGA
ATACTGCTACAACCCCAGCCACAACCCAGAGGAGCAGCTCTCCTCCAAGGACCTGGTGTCCTGC
GCCTACCAGGTGGCCCGAGGCATGGAGTATCTGGCCTCCAAGAAGTGCATACACCGAGACCTG
GCAGCCAGGAATGTCCTGGTGACAGAGGACAATGTGATGAAGATAGCAGACTTTGGCCTCGCA
CGGGACATTCACCACATCGACTACTATAAAAAGACAACCAACGGCCGACTGCCTGTGAAGTGG
ATGGCACCCGAGGCATTATTTGACCGGATCTACACCCACCAGAGTGATGTGTGGTCTTTCGGG
GTGCTCCTGTGGGAGATCTTCACTCTGGGCGGCTCCCCATACCCCGGTGTGCCTGTGGAGGAA
CTTTTCAAGCTGCTGAAGGAGGGTCACCGCATGGACAAGCCCAGTAACTGCACCAACGAGCTG
TACATGATGATGCGGGACTGCTGGCATGCAGTGCCCTCACAGAGACCCACCTTCAAGCAGCTG
GTGGAAGACCTGGACCGCATCGTGGCCTTGACCTCCAACCAGGAGTACCTGGACCTGTCCATG
CCCCTGGACCAGTACTCCCCCAGCTTTCCCGACACCCGGAGCTCTACGTGCTCCTCAGGGGAGG

ATTCCGTCTTCTCTCATGAGCCGCTGCCCGAGGAGCCCTGCCTGCCCCGACACCCAGCCCAGCT
TGCCAATGGCGGACTCAAACGCCGCTGA

The primers used for cloning of SEQ ID Nos: 15 and 16 are shown as SEQ ID Nos: 17 to 18 below.
SEQ ID No: 17 - TTGGACATCCCCAGAAAAGA (216_attB1_p68)
SEQ ID No: 18 - CCATATTGGACATCCCCAGA (217_attB1_p68)

Translation of SEQ ID No: 16 generates a protein comprising the amino acid sequence of SEQ ID No: 19.
SEQ ID No: 19

MEKKLHAVPAAKTVKFKCPSSGTPNPTLRWLKNGKEFKPDHRIGGYKVRYATWSIIMDSVVPS
DKGNYTCIVENEYGSINHTYQLDVVERSPHRPILQAGLPANKTVALGSNVEFMCKVYSDPQPHI
QWLKHIEVNGSKIGPDNLPYVQILKTAGVNTTDKEMEVLHLRNVSFEDAGEYTCLAGNSIGLS
HHSAWLTVLEALEERPAVMTSPLYLEIIIYCTGAFLISCMVGSVIVYKMKSGTKKSDFHSQMAV
HKLAKSIPLRRQVSADSSASMNSGVLLVRPSRLSSSGTPMLAGVSEYELPEDPRWELPRDRLVL
GKPLGEGCFGQVVLAEAIGLDKDKPNRVTKVAVKMLKSDATEKDLSDLISEMEMMKMIGKHK
NIINLLGACTQDGPLYVIVEYASKGNLREYLQARRPPGLEYCYNPSHNPEEQLSSKDLVSCAYQV
ARGMEYLASKKCIHRDLAARNVLVTEDNVMKIADFGLARDIHHIDYYKKTTNGRLPVKWMAP
EALFDRIYTHQSDVWSFGVLLWEIFTLGGSPYPGVPVEELFKLLKEGHRMDKPSNCTNELYMM
MRDCWHAVPSQRPTFKQLVEDLDRIVALTSNQEYLDLSMPLDQYSPSFPDTRSSTCSSGEDSVF
SHEPLPEEPCLPRHPAQLANGGLKRR

The altered FGFR1 protein comprising the amino acid sequence of SEQ ID No: 19 has 660 amino acids comprising two IG-like-domains, and the protein kinase domain.

SEQ ID No: 20 shows the transcript of sample A1116. Of note, a break within the 5' UTR (intron 1 of *FGFR1*) results in deletion of a multitude of regulatory elements on DNA level. On mRNA level, a deletion of the 5' UTR (coded from exon 1 of *FGFR1*) can be observed. SEQ ID No: 20 comprises the ATG start codon of wt-FGFRi coded in exon 2. Translation of the transcript with SEQ ID No: 20 corresponds to wt-FGFRi (SEQ ID No: 1; FGFR1 NM_015850).
SEQ ID No: 20

ACTGCTGGAGTTAATACCACCGACAAAGAGATGGAGGTGCTTCACTTAAGAAATGTCTCCTTT
GAGGACGCAGGGGAGTATACGTGCTTGGCGGGTAACTCTATCGGACTCTCCCATCACTCTGCA
TGGTTGACCGTTCTGGAAGCCCTGGAAGAGAGGCCGGCAGTGATGACCTCGCCCCTGTACCTG

GAGATCATCATCTATTGCACAGGGGCCTTCCTCATCTCCTGCATGGTGGGGTCGGTCATCGTCT
ACAAGATGAAGAGTGGTACCAAGAAGAGTGACTTCCACAGCCAGATGGCTGTGCACAAGCTGG
CCAAGAGCATCCCTCTGCGCAGACAGGTAACAGTGTCTGCTGACTCCAGTGCATCCATGAACTC
TGGGGTTCTTCTGGTTCGGCCATCACGGCTCTCCTCCAGTGGGACTCCCATGCTAGCAGGGGT
CTCTGAGTATGAGCTTCCCGAAGACCCTCGCTGGGAGCTGCCTCGGGACAGACTGGTCTTAGG
CAAACCCCTGGGAGAGGGCTGCTTTGGGCAGGTGGTGTTGGCAGAGGCTATCGGGCTGGACA
AGGACAAACCCAACCGTGTGACCAAAGTGGCTGTGAAGATGTTGAAGTCGGACGCAACAGAGA
AAGACTTGTCAGACCTGATCTCAGAAATGGAGATGATGAAGATGATCGGGAAGCATAAGAATA
TCATCAACCTGCTGGGGGCCTGCACGCAGGATGGTCCCTTGTATGTCATCGTGGAGTATGCCT
CCAAGGGCAACCTGCGGGAGTACCTGCAGGCCCGGAGGCCCCCAGGGCTGGAATACTGCTACA
ACCCCAGCCACAACCCAGAGGAGCAGCTCTCCTCCAAGGACCTGGTGTCCTGCGCCTACCAGG
TGGCCCGAGGCATGGAGTATCTGGCCTCCAAGAAGTGCATACACCGAGACCTGGCAGCCAGGA
ATGTCCTGGTGACAGAGGACAATGTGATGAAGATAGCAGACTTTGGCCTCGCACGGGACATTC
ACCACATCGACTACTATAAAAAGACAACCAACGGCCGACTGCCTGTGAAGTGGATGGCACCCG
AGGCATTATTTGACCGGATCTACACCCACCAGAGTGATGTGTGGTCTTTCGGGGTGCTCCTGT
GGGAGATCTTCACTCTGGGCGGCTCCCCATACCCCGGTGTGCCTGTGGAGGAACTTTTCAAGC
TGCTGAAGGAGGGTCACCGCATGGACAAGCCCAGTAACTGCACCAACGAGCTGTACATGATGA
TGCGGGACTGCTGGCATGCAGTGCCCTCACAGAGACCCACCTTCAAGCAGCTGGTGGAAGACC
TGGACCGCATCGTGGCCTTGACCTCCAACCAGGAGTACCTGGACCTGTCCATGCCCCTGGACC
AGTACTCCCCCAGCTTTCCCGACACCCGGAGCTCTACGTGCTCCTCAGGGGAGGATTCCGTCTT
CTCTCATGAGCCGCTGCCCGAGGAGCCCTGCCTGCCCCGACACCCAGCCCAGCTTGCCAATGG
CGGACTCAAACGCCGCTGACTGCCACCCACACGCCCTCCCCAGACTCCACCGTCAGCTGTAACC
CTCACCCACAGCCCCTGCTGGGCCCACCACCTGTCCGTCCCTGTCCCCTTTCCTGCTGGCAGGA
GCCGGCTGCCTACCAGGGGCCTTCCTGTGTGGCCTGCCTTCACCCCACTCAGCTCACCTCTCCC
TCCACCTCCTCTCCACCTGCTGGTGAGAGGTGCAAAGAGGCAGATCTTTGCTGCCAGCCACTTC
ATCCCCTCCCAGATGTTGGACCAACACCCCTCCCTGCCACCAGGCACTGCCTGGAGGGCAGGG
AGTGGGAGCCAATGAACAGGCATGCAAGTGAGAGCTTCCTGAGCTTTCTCCTGTCGGTTTGGT
CTGTTTTGCCTTCACCCATAAGCCCCTCGCACTCTGGTGGCAGGTGCCTTGTCCTCAGGGCTAC
AGCAGTAGGGAGGTCAGTGCTTCGTGCCTCGATTGAAGGTGACCTCTGCCCCAGATAGGTGGT
GCCAGTGGCTTATTAATTCCGATACTAGTTTGCTTTGCTGACCAAATGCCTGGTACCAGAGGAT
GGTGAGGCGAAGGCCAGGTTGGGGGCAGTGTTGTGGCCCTGGGGCCCAGCCCCAAACTGGGG
GCTCTGTATATAGCTATGAAGAAAACACAAAGTGTATAAATCTGAGTATATATTTACATGTCTT
TTTAAAAGGGTCGTTACCAGAGATTTACCCATCGGGTAAGATGCTCCTGGTGGCTGGGAGGCA
TCAGTTGCTATATATTAAAAACAAAAAAGAAAAAAAAGGAAAATGTTTTTAAAAAGGTCATATA
TTTTTTGCTACTTTTGCTGTTTTATTTTTTTAAATTATGTTCTAAACCTATTTTCAGTTTAGGTCC
CTCAATAAAAATTGCTGCTGCTTCA

The underlined part in SEQ ID No: 20 is being transcribed and is shown as SEQ ID No: 21 below.
SEQ ID No: 21

ATGGAGGTGCTTCACTTAAGAAATGTCTCCTTTGAGGACGCAGGGGAGTATACGTGCTTGGCG
GGTAACTCTATCGGACTCTCCCATCACTCTGCATGGTTGACCGTTCTGGAAGCCCTGGAAGAGA
GGCCGGCAGTGATGACCTCGCCCCTGTACCTGGAGATCATCATCTATTGCACAGGGGCCTTCCT
CATCTCCTGCATGGTGGGGTCGGTCATCGTCTACAAGATGAAGAGTGGTACCAAGAAGAGTGA
CTTCCACAGCCAGATGGCTGTGCACAAGCTGGCCAAGAGCATCCCTCTGCGCAGACAGGTAAC
AGTGTCTGCTGACTCCAGTGCATCCATGAACTCTGGGGTTCTTCTGGTTCGGCCATCACGGCTC
TCCTCCAGTGGGACTCCCATGCTAGCAGGGGTCTCTGAGTATGAGCTTCCCGAAGACCCTCGCT
GGGAGCTGCCTCGGGACAGACTGGTCTTAGGCAAACCCCTGGGAGAGGGCTGCTTTGGGCAG
GTGGTGTTGGCAGAGGCTATCGGGCTGGACAAGGACAAACCCAACCGTGTGACCAAAGTGGC
TGTGAAGATGTTGAAGTCGGACGCAACAGAGAAAGACTTGTCAGACCTGATCTCAGAAATGGA
GATGATGAAGATGATCGGGAAGCATAAGAATATCATCAACCTGCTGGGGGCCTGCACGCAGGA
TGGTCCCTTGTATGTCATCGTGGAGTATGCCTCCAAGGGCAACCTGCGGGAGTACCTGCAGGC
CCGGAGGCCCCCAGGGCTGGAATACTGCTACAACCCCAGCCACAACCCAGAGGAGCAGCTCTC
CTCCAAGGACCTGGTGTCCTGCGCCTACCAGGTGGCCCGAGGCATGGAGTATCTGGCCTCCAA
GAAGTGCATACACCGAGACCTGGCAGCCAGGAATGTCCTGGTGACAGAGGACAATGTGATGAA
GATAGCAGACTTTGGCCTCGCACGGGACATTCACCACATCGACTACTATAAAAAGACAACCAAC
GGCCGACTGCCTGTGAAGTGGATGGCACCCGAGGCATTATTTGACCGGATCTACACCCACCAG
AGTGATGTGTGGTCTTTCGGGGTGCTCCTGTGGGAGATCTTCACTCTGGGCGGCTCCCCATAC
CCCGGTGTGCCTGTGGAGGAACTTTTCAAGCTGCTGAAGGAGGGTCACCGCATGGACAAGCCC
AGTAACTGCACCAACGAGCTGTACATGATGATGCGGGACTGCTGGCATGCAGTGCCCTCACAG
AGACCCACCTTCAAGCAGCTGGTGGAAGACCTGGACCGCATCGTGGCCTTGACCTCCAACCAG
GAGTACCTGGACCTGTCCATGCCCCTGGACCAGTACTCCCCCAGCTTTCCCGACACCCGGAGCT
CTACGTGCTCCTCAGGGGAGGATTCCGTCTTCTCTCATGAGCCGCTGCCCGAGGAGCCCTGCCT
GCCCCGACACCCAGCCCAGCTTGCCAATGGCGGACTCAAACGCCGCTGA

The primers used for cloning of SEQ ID Nos: 20 and 21 are shown as SEQ ID Nos: 22 to 23 below.
SEQ ID No: 22 - CCGACAAAGAGATGGAGGTG (218_attB1_FGFR1_Virtuell)
SEQ ID No: 23 - CAAAGAGATGGAGGTGCTTCAC (219_attB1_FGFR1_Virtuell)

SEQ ID No: 25 is the FGFR1 transcript of a further exemplary sample of a responder to FGFRi-inhibitor therapy.
SEQ ID No: 25

ATGGTGGGGTCGGTCATCGTCTACAAGATGAAGAGTGGTACCAAGAAGAGTGACTTCC
ACAGCCAGATGGCTGTGCACAAGCTGGCCAAGAGCATCCCTCTGCGCAGACAGGTGTCTGCTG
ACTCCAGTGCATCCATGAACTCTGGGGTTCTTCTGGTTCGGCCATCACGGCTCTCCTCCAGTGG
GACTCCCATGCTAGCAGGGGTCTCTGAGTATGAGCTTCCCGAAGACCCTCGCTGGGAGCTGCC
TCGGGACAGACTGGTCTTAGGCAAACCCCTGGGAGAGGGCTGCTTTGGGCAGGTGGTGTTGG
CAGAGGCTATCGGGCTGGACAAGGACAAACCCAACCGTGTGACCAAAGTGGCTGTGAAGATGT
TGAAGTCGGACGCAACAGAGAAAGACTTGTCAGACCTGATCTCAGAAATGGAGATGATGAAGA
TGATCGGGAAGCATAAGAATATCATCAACCTGCTGGGGGCCTGCACGCAGGATGGTCCCTTGT
ATGTCATCGTGGAGTATGCCTCCAAGGGCAACCTGCGGGAGTACCTGCAGGCCCGGAGGCCCC
CAGGGCTGGAATACTGCTACAACCCCAGCCACAACCCAGAGGAGCAGCTCTCCTCCAAGGACC
TGGTGTCCTGCGCCTACCAGGTGGCCCGAGGCATGGAGTATCTGGCCTCCAAGAAGTGCATAC
ACCGAGACCTGGCAGCCAGGAATGTCCTGGTGACAGAGGACAATGTGATGAAGATAGCAGACT
TTGGCCTCGCACGGGACATTCACCACATCGACTACTATAAAAAGACAACCAACGGCCGACTGCC
TGTGAAGTGGATGGCACCCGAGGCATTATTTGACCGGATCTACACCCACCAGAGTGATGTGTG
GTCTTTCGGGGTGCTCCTGTGGGAGATCTTCACTCTGGGCGGCTCCCCATACCCCGGTGTGCCT
GTGGAGGAACTTTTCAAGCTGCTGAAGGAGGGTCACCGCATGGACAAGCCCAGTAACTGCACC
AACGAGCTGTACATGATGATGCGGGACTGCTGGCATGCAGTGCCCTCACAGAGACCCACCTTC
AAGCAGCTGGTGGAAGACCTGGACCGCATCGTGGCCTTGACCTCCAACCAGGAGTACCTGGAC
CTGTCCATGCCCCTGGACCAGTACTCCCCCAGCTTTCCCGACACCCGGAGCTCTACGTGCTCCT
CAGGGGAGGATTCCGTCTTCTCTCATGAGCCGCTGCCCGAGGAGCCCTGCCTGCCCCGACACC
CAGCCCAGCTTGCCAATGGCGGACTCAAACGCCGCTGA

Translation of SEQ ID No: 25 generates a protein comprising the amino acid sequence of SEQ ID No: 26.
SEQ ID No: 26

The altered FGFR1 protein comprising the amino acid sequence of SEQ ID No: 26 lacks additional 21 amino acids due to the N-terminal truncation when compared to ΔEC-FGFR1 (SEQ ID No: 14). SEQ ID No: 26 is also referred to as ΔEC-21-FGFR1. The double-underlined residues in SEQ ID No: 26 are part of a shorter transmembrane domain, whereas the single-underlined residues form the kinase domain.

The forward primer used for cloning of SEQ ID No: 25 is shown as SEQ ID No: 27 below. SEQ ID No: 27 - ATGGTGGGGTCGGTCAT (230_F_ΔEC-21-FGFR1). As reverse primer for cloning of SEQ ID No: 25, any reverse primer in exon 1 to 12 of *FGFR1* can be used.

SEQ ID No: 28 is the FGFR1 transcript of a further exemplary sample of a responder to FGFRi-inhibitor therapy.
SEQ ID No: 28

ATGAAGAGTGGTACCAAGAAGAGTGACTTCCACAGCCAGATGGCTGTGCACAAGCTGGCCAAG
AGCATCCCTCTGCGCAGACAGGTGTCTGCTGACTCCAGTGCATCCATGAACTCTGGGGTTCTTC
TGGTTCGGCCATCACGGCTCTCCTCCAGTGGGACTCCCATGCTAGCAGGGGTCTCTGAGTATG
AGCTTCCCGAAGACCCTCGCTGGGAGCTGCCTCGGGACAGACTGGTCTTAGGCAAACCCCTGG
GAGAGGGCTGCTTTGGGCAGGTGGTGTTGGCAGAGGCTATCGGGCTGGACAAGGACAAACCC
AACCGTGTGACCAAAGTGGCTGTGAAGATGTTGAAGTCGGACGCAACAGAGAAAGACTTGTCA
GACCTGATCTCAGAAATGGAGATGATGAAGATGATCGGGAAGCATAAGAATATCATCAACCTG
CTGGGGGCCTGCACGCAGGATGGTCCCTTGTATGTCATCGTGGAGTATGCCTCCAAGGGCAAC
CTGCGGGAGTACCTGCAGGCCCGGAGGCCCCCAGGGCTGGAATACTGCTACAACCCCAGCCAC
AACCCAGAGGAGCAGCTCTCCTCCAAGGACCTGGTGTCCTGCGCCTACCAGGTGGCCCGAGGC
ATGGAGTATCTGGCCTCCAAGAAGTGCATACACCGAGACCTGGCAGCCAGGAATGTCCTGGTG
ACAGAGGACAATGTGATGAAGATAGCAGACTTTGGCCTCGCACGGGACATTCACCACATCGAC
TACTATAAAAAGACAACCAACGGCCGACTGCCTGTGAAGTGGATGGCACCCGAGGCATTATTT
GACCGGATCTACACCCACCAGAGTGATGTGTGGTCTTTCGGGGTGCTCCTGTGGGAGATCTTC
ACTCTGGGCGGCTCCCCATACCCCGGTGTGCCTGTGGAGGAACTTTTCAAGCTGCTGAAGGAG
GGTCACCGCATGGACAAGCCCAGTAACTGCACCAACGAGCTGTACATGATGATGCGGGACTGC
TGGCATGCAGTGCCCTCACAGAGACCCACCTTCAAGCAGCTGGTGGAAGACCTGGACCGCATC
GTGGCCTTGACCTCCAACCAGGAGTACCTGGACCTGTCCATGCCCCTGGACCAGTACTCCCCCA
GCTTTCCCGACACCCGGAGCTCTACGTGCTCCTCAGGGGAGGATTCCGTCTTCTCTCATGAGCC
GCTGCCCGAGGAGCCCTGCCTGCCCCGACACCCAGCCCAGCTTGCCAATGGCGGACTCAAACG
CCGCTGA

Translation of SEQ ID No: 28 generates a protein comprising the amino acid sequence of SEQ ID No: 29.
SEQ ID No: 29

MKSGTKKSDFHSQMAVHKLAKSIPLRRQVSADSSASMNSGVLLVRPSRLSSSGTPMLAGVSEYE
LPEDPRWELPRDRLVLGKPLGEGCFGQVVLAEAIGLDKDKPNRVTKVAVKMLKSDATEKDLSD
LISEMEMMKMIGKHKNIINLLGACTQDGPLYVIVEYASKGNLREYLQARRPPGLEYCYNPSHNP
EEQLSSKDLVSCAYQVARGMEYLASKKCIHRDLAARNVLVTEDNVMKIADFGLARDIHHIDYY
KKTTNGRLPVKWMAPEALFDRIYTHQSDVWSFGVLLWEIFTLGGSPYPGVPVEELFKLLKEGH
RMDKPSNCTNELYMMMRDCWHAVPSQRPTFKQLVEDLDRIVALTSNQEYLDLSMPLDQYSPS
FPDTRSSTCSSGEDSVFSHEPLPEEPCLPRHPAQLANGGLKRR

The altered FGFR1 protein comprising the amino acid sequence of SEQ ID No: 29 lacks 30 amino acids when compared to ΔEC-FGFR1 (SEQ ID No: 14). SEQ ID No: 29 is also referred to as ΔEC-30-FGFR1. Due to the N-terminal truncation, the protein comprising the amino acid sequence of SEQ ID No: 29 has no IG-like domain, but a kinase domain. The underlined residues in SEQ ID No: 29 above form the kinase domain.

The forward primer used for cloning of SEQ ID No: 28 is shown as SEQ ID No: 30 below. SEQ ID No: 30 - ATGAAGAGTGGTACCAAGAAGAGTG (231_F_ΔEC-30-FGFR1). As reverse primer for cloning of SEQ ID No: 28, any reverse primer in exon 1 to 12 of *FGFR1* can be used.

SEQ ID No: 31 is the FGFR1 transcript of a further exemplary sample of a responder to FGFRi-inhibitor therapy.
SEQ ID No: 31

ATGCTAGCAGGGGTCTCTGAGTATGAGCTTCCCGAAGACCCTCGCTGGGAGCTGCCTCGGGAC
AGACTGGTCTTAGGCAAACCCCTGGGAGAGGGCTGCTTTGGGCAGGTGGTGTTGGCAGAGGC
TATCGGGCTGGACAAGGACAAACCCAACCGTGTGACCAAAGTGGCTGTGAAGATGTTGAAGTC
GGACGCAACAGAGAAAGACTTGTCAGACCTGATCTCAGAAATGGAGATGATGAAGATGATCGG
GAAGCATAAGAATATCATCAACCTGCTGGGGGCCTGCACGCAGGATGGTCCCTTGTATGTCAT
CGTGGAGTATGCCTCCAAGGGCAACCTGCGGGAGTACCTGCAGGCCCGGAGGCCCCCAGGGC
TGGAATACTGCTACAACCCCAGCCACAACCCAGAGGAGCAGCTCTCCTCCAAGGACCTGGTGT
CCTGCGCCTACCAGGTGGCCCGAGGCATGGAGTATCTGGCCTCCAAGAAGTGCATACACCGAG
ACCTGGCAGCCAGGAATGTCCTGGTGACAGAGGACAATGTGATGAAGATAGCAGACTTTGGCC
TCGCACGGGACATTCACCACATCGACTACTATAAAAAGACAACCAACGGCCGACTGCCTGTGA
AGTGGATGGCACCCGAGGCATTATTTGACCGGATCTACACCCACCAGAGTGATGTGTGGTCTT
TCGGGGTGCTCCTGTGGGAGATCTTCACTCTGGGCGGCTCCCCATACCCCGGTGTGCCTGTGG
AGGAACTTTTCAAGCTGCTGAAGGAGGGTCACCGCATGGACAAGCCCAGTAACTGCACCAACG
AGCTGTACATGATGATGCGGGACTGCTGGCATGCAGTGCCCTCACAGAGACCCACCTTCAAGC
AGCTGGTGGAAGACCTGGACCGCATCGTGGCCTTGACCTCCAACCAGGAGTACCTGGACCTGT
CCATGCCCCTGGACCAGTACTCCCCCAGCTTTCCCGACACCCGGAGCTCTACGTGCTCCTCAGG

GGAGGATTCCGTCTTCTCTCATGAGCCGCTGCCCGAGGAGCCCTGCCTGCCCCGACACCCAGC
CCAGCTTGCCAATGGCGGACTCAAACGCCGCTGA

Translation of SEQ ID No: 31 generates a protein comprising the amino acid sequence of SEQ ID No: 32.
SEQ ID No: 32

MLAGVSEYELPEDPRWELPRDRLVLGKPLGEGCFGQVVLAEAIGLDKDKPNRVTKVAVKMLKS
DATEKDLSDLISEMEMMKMIGKHKNIINLLGACTQDGPLYVIVEYASKGNLREYLQARRPPGLE
YCYNPSHNPEEQLSSKDLVSCAYQVARGMEYLASKKCIHRDLAARNVLVTEDNVMKIADFGLA
RDIHHIDYYKKTTNGRLPVKWMAPEALFDRIYTHQSDVWSFGVLLWEIFTLGGSPYPGVPVEE
LFKLLKEGHRMDKPSNCTNELYMMMRDCWHAVPSQRPTFKQLVEDLDRIVALTSNQEYLDLS
MPLDQYSPSFPDTRSSTCSSGEDSVFSHEPLPEEPCLPRHPAQLANGGLKRR

The altered FGFR1 protein comprising the amino acid sequence of SEQ ID No: 32 lacks 85 amino acids when compared to ΔEC-FGFR1 (SEQ ID No: 14). SEQ ID No: 32 is also referred to as ΔEC-85-FGFR1. The protein comprising the amino acid sequence of SEQ ID No: 32 has no IG-like domain due to the N-terminal truncation, but a kinase domain. The underlined residues in SEQ ID No: 32 form the kinase domain.

The forward primer used for cloning of SEQ ID No: 31 is shown as SEQ ID No: 33 below. SEQ ID No: 33 - CATGCTAGCAGGGGTCTCT (232_F_ΔEC-85-FGFR1). As reverse primer for cloning of SEQ ID No: 25, any reverse primer in exon 1 to 12 of *FGFR1* can be used.

SEQ ID No: 34 is the FGFR1 transcript of a further exemplary sample of a responder to FGFRi-inhibitor therapy.
SEQ ID No: 34

ATGCTAGCAGGGGTCTCTGAGTATGAGCTTCCCGAAGACCCTCGCTGGGAGCTGCCTCGGGAC
AGACTGGTCTTAGGCAAACCCCTGGGAGAGGGCTGCTTTGGGCAGGTGGTGTTGGCAGAGGC
TATCGGGCTGGACAAGGACAAACCCAACCGTGTGACCAAAGTGGCTGTGAAGATGTTGAAGTC
GGACGCAACAGAGAAAGACTTGTCAGACCTGATCTCAGAAATGGAGATGATGAAGATGATCGG
GAAGCATAAGAATATCATCAACCTGCTGGGGGCCTGCACGCAGGATGGTCCCTTGTATGTCAT
CGTGGAGTATGCCTCCAAGGGCAACCTGCGGGAGTACCTGCAGGCCCGGAGGCCCCCAGGGC
TGGAATACTGCTACAACCCCAGCCACAACCCAGAGGAGCAGCTCTCCTCCAAGGACCTGGTGT
CCTGCGCCTACCAGGTGGCCCGAGGCATGGAGTATCTGGCCTCCAAGAAGTGCATACACCGAG
ACCTGGCAGCCAGGAATGTCCTGGTGACAGAGGACAATGTGATGAAGATAGCAGACTTTGGCC
TCGCACGGGACATTCACCACATCGACTACTATAAAAAGACAACCAACGGCCGACTGCCTGTGA

AGTGGATGGCACCCGAGGCATTATTTGACCGGATCTACACCCACCAGAGTGATGTGTGGTCTT
TCGGGGTGCTCCTGTGGGAGATCTTCACTCTGGGCGGCTCCCCATACCCCGGTGTGCCTGTGG
AGGAACTTTTCAAGCTGCTGAAGGAGGGTCACCGCATGGACAAGCCCAGTAACTGCACCAACG
AGCTGTACATGATGATGCGGGACTGCTGGCATGCAGTGCCCTCACAGAGACCCACCTTCAAGC
AGCTGGTGGAAGACCTGGACCGCATCGTGGCCTTGACCTCCAACCAGGAGTACCTGGACCTGT
CCATGCCCCTGGACCAGTACTCCCCCAGCTTTCCCGACACCCGGAGCTCTACGTGCTCCTCAGG
GGAGGATTCCGTCTTCTCTCATGAGCCGCTGCCCGAGGAGCCCTGCCTGCCCCGACACCCAGC
CCAGCTTGCCAATGGCGGACTCAAACGCCGCTGA

Translation of SEQ ID No: 34 generates a protein comprising the amino acid sequence of SEQ ID No: 35.
SEQ ID No: 35

MLKSDATEKDLSDLISEMEMMKMIGKHKNIINLLGACTQDGPLYVIVEYASKGNLREYLQARR
PPGLEYCYNPSHNPEEQLSSKDLVSCAYQVARGMEYLASKKCIHRDLAARNVLVTEDNVMKIA
DFGLARDIHHIDYYKKTTNGRLPVKWMAPEALFDRIYTHQSDVWSFGVLLWEIFTLGGSPYPG
VPVEELFKLLKEGHRMDKPSNCTNELYMMMRDCWHAVPSQRPTFKQLVEDLDRIVALTSNQE
YLDLSMPLDQYSPSFPDTRSSTCSSGEDSVFSHEPLPEEPCLPRHPAQLANGGLKRR

The altered FGFR1 protein comprising the amino acid sequence of SEQ ID No: 35 lacks 144 amino acids when compared to ΔEC-FGFR1 (SEQ ID No: 14). Therefore, SEQ ID No: 35 is also referred to as ΔEC-144-FGFR1. Due to the N-terminal truncation, the protein comprising the amino acid sequence of SEQ ID No: 35 has no IG-like domain, and most likely no functional kinase domain. However, residues that form a part of the kinase domain of the amino acid sequence of SEQ ID No: 35 are underlined.

The forward primer used for cloning of SEQ ID No: 34 is shown as SEQ ID No: 36 below. SEQ ID No: 36 - ATGCTAGCAGGGGTCTCTGA (233_F_ΔEC-144-FGFR1). As reverse primer for cloning of SEQ ID No: 25, any reverse primer in exon 1 to 12 of *FGFR1* can be used.

SEQ ID No: 37 shows wild type FGFR1 (NM_015850)
SEQ ID No: 37

ATGTGGAGCTGGAAGTGCCTCCTCTTCTGGGCTGTGCTGGTCACAGCCACACTCTGCAC
CGCTAGGCCGTCCCCGACCTTGCCTGAACAAGCCCAGCCCTGGGGAGCCCCTGTGGAAGTGGA
GTCCTTCCTGGTCCACCCCGGTGACCTGCTGCAGCTTCGCTGTCGGCTGCGGGACGATGTGCA
GAGCATCAACTGGCTGCGGGACGGGGTGCAGCTGGCGGAAAGCAACCGCACCCGCATCACAG
GGGAGGAGGTGGAGGTGCAGGACTCCGTGCCCGCAGACTCCGGCCTCTATGCTTGCGTAACCA
GCAGCCCCTCGGGCAGTGACACCACCTACTTCTCCGTCAATGTTTCAGATGCTCTCCCCTCCTC

GGAGGATGATGATGATGATGATGACTCCTCTTCAGAGGAGAAAGAAACAGATAACACCAAACC
AAACCCCGTAGCTCCATATTGGACATCCCCAGAAAAGATGGAAAAGAAATTGCATGCAGTGCC
GGCTGCCAAGACAGTGAAGTTCAAATGCCCTTCCAGTGGGACCCCAAACCCCACACTGCGCTG
GTTGAAAAATGGCAAAGAATTCAAACCTGACCACAGAATTGGAGGCTACAAGGTCCGTTATGC
CACCTGGAGCATCATAATGGACTCTGTGGTGCCCTCTGACAAGGGCAACTACACCTGCATTGT
GGAGAATGAGTACGGCAGCATCAACCACACATACCAGCTGGATGTCGTGGAGCGGTCCCCTCA
CCGGCCCATCCTGCAAGCAGGGTTGCCCGCCAACAAAACAGTGGCCCTGGGTAGCAACGTGGA
GTTCATGTGTAAGGTGTACAGTGACCCGCAGCCGCACATCCAGTGGCTAAAGCACATCGAGGT
GAATGGGAGCAAGATTGGCCCAGACAACCTGCCTTATGTCCAGATCTTGAAGACTGCTGGAGT
TAATACCACCGACAAAGAGATGGAGGTGCTTCACTTAAGAAATGTCTCCTTTGAGGACGCAGG
GGAGTATACGTGCTTGGCGGGTAACTCTATCGGACTCTCCCATCACTCTGCATGGTTGACCGTT
CTGGAAGCCCTGGAAGAGAGGCCGGCAGTGATGACCTCGCCCCTGTACCTGGAGATCATCATC
TATTGCACAGGGGCCTTCCTCATCTCCTGCATGGTGGGGTCGGTCATCGTCTACAAGATGAAG
AGTGGTACCAAGAAGAGTGACTTCCACAGCCAGATGGCTGTGCACAAGCTGGCCAAGAGCATC
CCTCTGCGCAGACAGGTAACAGTGTCTGCTGACTCCAGTGCATCCATGAACTCTGGGGTTCTTC
TGGTTCGGCCATCACGGCTCTCCTCCAGTGGGACTCCCATGCTAGCAGGGGTCTCTGAGTATG
AGCTTCCCGAAGACCCTCGCTGGGAGCTGCCTCGGGACAGACTGGTCTTAGGCAAACCCCTGG
GAGAGGGCTGCTTTGGGCAGGTGGTGTTGGCAGAGGCTATCGGGCTGGACAAGGACAAACCC
AACCGTGTGACCAAAGTGGCTGTGAAGATGTTGAAGTCGGACGCAACAGAGAAAGACTTGTCA
GACCTGATCTCAGAAATGGAGATGATGAAGATGATCGGGAAGCATAAGAATATCATCAACCTG
CTGGGGGCCTGCACGCAGGATGGTCCCTTGTATGTCATCGTGGAGTATGCCTCCAAGGGCAAC
CTGCGGGAGTACCTGCAGGCCCGGAGGCCCCCAGGGCTGGAATACTGCTACAACCCCAGCCAC
AACCCAGAGGAGCAGCTCTCCTCCAAGGACCTGGTGTCCTGCGCCTACCAGGTGGCCCGAGGC
ATGGAGTATCTGGCCTCCAAGAAGTGCATACACCGAGACCTGGCAGCCAGGAATGTCCTGGTG
ACAGAGGACAATGTGATGAAGATAGCAGACTTTGGCCTCGCACGGGACATTCACCACATCGAC
TACTATAAAAAGACAACCAACGGCCGACTGCCTGTGAAGTGGATGGCACCCGAGGCATTATTT
GACCGGATCTACACCCACCAGAGTGATGTGTGGTCTTTCGGGGTGCTCCTGTGGGAGATCTTC
ACTCTGGGCGGCTCCCCATACCCCGGTGTGCCTGTGGAGGAACTTTTCAAGCTGCTGAAGGAG
GGTCACCGCATGGACAAGCCCAGTAACTGCACCAACGAGCTGTACATGATGATGCGGGACTGC
TGGCATGCAGTGCCCTCACAGAGACCCACCTTCAAGCAGCTGGTGGAAGACCTGGACCGCATC
GTGGCCTTGACCTCCAACCAGGAGTACCTGGACCTGTCCATGCCCCTGGACCAGTACTCCCCCA
GCTTTCCCGACACCCGGAGCTCTACGTGCTCCTCAGGGGAGGATTCCGTCTTCTCTCATGAGCC
GCTGCCCGAGGAGCCCTGCCTGCCCCGACACCCAGCCCAGCTTGCCAATGGCGGACTCAAACG
CCGCTGA

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Identification of head-to-head followed by tail-to-tail intra-chromosomal breaks within the 8p arm by a Breakage-Fusion-Bridge-like mechanism in squamous cell lung cancer

The inventors analyzed whether a distinct 8p11 amplified genome structure, such as heterogeneous 8p11-8p12 amplification patterns, leads to genotypic and phenotypic *FGFR1-*dependency, i.e. the responsiveness of *FGFR1*-amplified tumors to FGFR inhibitor (FGFRi) therapy. Specifically, the inventors first analyzed 8p11-12-amplified squamous cell lung carcinomas with unknown responsiveness to FGFR inhibition.

**Human lung tumor specimens (initial study cohort):** The inventors collected in total 27 fresh-frozen squamous cell lung cancer tumor (SQLC) samples, which were provided by multiple collaborating institutions as fresh-frozen tissue specimen, frozen sections or as genomic DNA extracted from fresh-frozen material. All tumor samples were pathologically assessed to have a purity of at least 60% and no extensive signs of necrosis. Additionally, these tumor samples were reviewed by at least two independent expert pathologists and the diagnosis of SQLC was histomorphologically confirmed by H&E staining and immunohistochemistry. For immunohistochemistry, tissues were fixed in 4% PBS-buffered formalin and embedded in paraffin (FFPE). Immunohistochemistry was performed as described previously on 3 µm slides with specific antibodies for pFGFRi (Abnova, Y154).

Matching normal material was provided in the form of EDTA-anticoagulated blood or adjacent non-tumorigenic lung tissue. The matched normal tissue was confirmed to be free of tumor contaminants by pathological assessment. Furthermore, tumor and matching normal material were confirmed to be acquired from the same patient by SNP 6.0 array and sequencing analyses. Human tumour samples were obtained from patients under IRB-approved protocols following written informed consent and Patient material was stored at -80 °C.

**Genome and transcriptome sequencing:** The data is processed by aligning sequencing reads to a reference genome (NCBI build 37/hg19) using bwa-mem (0.7.13-r1126; https://github.com/1h3/bwa), masking potential PCR-duplicates and regions of overlapping read pairs, and the collection of various count statistics which are used to call mutations and, for whole genomes, genomic rearrangements. Finally, Sclust is used for sample purity estimation and copy number analysis. RNA-seq data was analysed using TRUP.

**ARCHER sequencing:** RNA was extracted from FFPE material using the Maxwell RSC in combination with the Maxwell RSC RNA FFPE Kit (Promega) according to the manufacturer's instruction. Removal of genomic DNA was performed with the TURBO DNA-free Kit (Thermo Fisher Scientific) and both tRNA and RNA were quantified using the Qubit RNA HS Assay Kit (Thermo Fisher). Analysis of RNA integrity was done with the 4200 TapeStation System (Agilent Technologies, Santa Clara, CA, USA).

For fusion detection, the Archer FusionPlex Lung Panel (ArcherDX) was used according to manufacturer's instructions with 200 ng tRNA input for library preparation. Using the KAPA Library Quantification Kit (Roche, Pleasanton, CA, USA), purified libraries were quantified. Sequencing was performed on the Nextseq System (Illumina, San Diego, CA, USA) and results were analyzed with the Archer Suite Analysis v 6.2.7 (ArcherDX). Since the Archer Suite Analysis software is not able to label inversions, bam files were extracted and loaded directly in the Integrative Genomics Viewer (IGV) to visualize tail-to-tail breaks.

**Computational Analysis:** Copy numbers were visualized with GISTIC (Ref. GISTIC2.0 facilitates sensitive and confident localization of the targets of focal somatic copy-number alteration in human cancers) and ROBOCOP. To summaries the copy number information in the FGFR1 region across samples, a view window of 2MB around the FGFR1 locus was split into 10'000 bins. The available chromosome segment-based copy number data were then mapped onto these bins for each of the samples. Next, the per-bin means of the copy numbers for all samples within a certain condition were calculated and the rolling mean with a window size of 10 (for whole genome sequencing data) and 500 of these numbers was determined to reduce noise in the CAGE data. The process of determining ROlling Binmeans Of COPy numbers is defined here as the ROBOCOP algorithm. Given a chromosome segmentation and sequence of segment copy number values n at the (amplified) FGFR1 locus, the approach estimates an approximate copy number sequence "n'" by applying palindromic BFB transformations to an initial wild-type segmentation. A similarity/distance between the sequences is measured as the Poisson likelihood of observing sequence n given the approximation n'. Using the provided Java application, BFB inference was done in two steps: (1) Estimating n' (java bfb.BFB_Algo counts:n mode:substring model:Poisson maxError:o) and (2) converting n' into a sequence of BFB transformations (java bfb.BFB_Algo counts:n' mode:search). Interestingly, only amplicons with a BFB approximation of at least eight segments were considered as high confidence (n=11), while amplicons with less than eight segments and/or no support by fold-back inversions were too ambiguous to reliably infer BFB as the underlying mechanism (n=14).

The inventors performed whole-genome sequencing and transcriptome (22 of 25) sequencing of tumors and their matched normal tissue. As expected, plotting the average copy number of all 25 samples revealed that the amplification was centered on *FGFR1* and the adjacent *NSD3* gene (FDR *q* = 1.3x10⁻³⁸) (**Fig. 1a**). In order to decode the precise genomic architecture of the 8p11-12 amplicon, the inventors inspected all occurring rearrangements and termed them according to their orientation, as compared to the reference genome (*normal, head-to-head* or *tail-to-tail*). The inventors found that only the amplicons with tail-to-tail breaks close to the transcriptional start side of *FGFR1* drove the amplification pattern that was centered around *FGFR1*/*NSD₃.* The inventors identified 9 samples, in which tail-to-tail breaks occurred within a 400-kilobase (kb) region upstream of *FGFR1* (36%). These samples exhibited a focal amplicon of 550 kb that was focused on only one gene, *FGFR1,* whereas the amplified region was three times larger (1.7 mb) in tumors without distinct tail-to-tail breaks (**Fig. 1a**). In 5 out of the 9 specimens (56%), in which the amplicon centered on *FGFR1,* a second break that led to the focal amplification, was within the gene *NSD3* (or *WHSC1L1*). These breaks were mainly head-to-head breaks (4 out of 5) and led to deletion of exons 13 to 23 of *NSD3* (**Fig. 1b**). Tumors with head-to-head breaks within *NSD3* exhibited increased expression of the short isoform of *NSD₃,* which lacks the catalytic SET domain and has been reported to be non-oncogenic. On the contrary, a tail-to-tail break within *FGFR1* occurred in another tumor amplifying particularly the intracellular part of FGFR1 (**Fig 1c**). The implications of this break will be described below (**Fig. 3**). Thus, in 9 of 25 8p-amplified squamous cell lung cancer specimens, tail-to-tail rearrangements caused focal amplifications that were centered exclusively on *FGFR1.* In 4 of these tumors, the inventors found predominant expression of a non-oncogenic version of *NSD3* lacking the SET domain-encoding exons resulting from deleterious genomic events (**Fig 1b**).

Further detailed analyses indicated that the pattern of genomic rearrangements on 8p originated from *Breakage-Fusion-Bridge* (BFB) cycles with characteristic features of telomeric loss, clipped read orientation, copy number variation and at least eight copy number segments (**Fig. 1d**). In all 25 *FGFR1*-amplified cases a telomeric loss was observed that was frequently accompanied by intra-chromosomal head-to-head and tail-to-tail fusions (detected as head-to-head or tail-to-tail breaks by clipped read orientation) (**Fig**. **1d**, **e**). The inventors were thus able to reliably identify BFBs as the underlying cause of 8p amplification in 44% (11 of 25) of the tumors. In 56% of the samples a BFB mechanism was uncertain (48%) or excluded (8%), because of low segmentation or no support from the observed rearrangements. Finally, again confirming a BFB mechanism, the inventors were able to correctly reconstruct the precise 8p amplicon in one tumor considering only the observed genomic breaks and following the assembly logic of BFB as the underlying mechanism (**Fig. 1f**, **g**). In summary, 8p amplifications in squamous cell lung cancer are caused by breakage fusion bridges in the majority of cases. Intra-chromosomal tail-to-tail breaks close to the *FGFR1* transcription start side followed by head-to-head breaks in or close to *NSD3* cause focal and *FGFR1*-centered amplification.

In summary, the inventors showed that 8p11-8p12 amplifications are driven by a BFB-like mechanism. Intra-chromosomal tail-to-tail breaks close to the *FGFR1* transcription start side cause focal amplification and are associated with FGFR inhibitor sensitivity.

### Example 2: Confirmation of a breakage-fusion-bridge mechanism forming 8p11-p12-amplified lung cancer tumors and the underlying mechanism of FGFR1-inhibitor dependency

To gain a deeper understanding of 8p11-8p12-amplified lung cancer tumors and to link specific patterns of the 8p12-8p11 amplicon structure with FGFR1 dependency, the inventors analyzed an independent cohort of 26 8p-amplified tumor specimens with known sensitivity to FGFR inhibition. In detail, the inventors analyzed 8 *FGFR1*-amplified lung cancer cell lines (**Fig. 2a****, b**), of which 6 were resistant to FGFR inhibition with the small-molecule inhibitors, BGJ398 and AZD4547 (half-growth-inhibitory concentration, or *GI₅₀,* > 1 µM) and two cell lines were sensitive (GI₅₀ < 1 µM, H1581 and DMS114) (**Fig. 2a**). Confirming the inventors' hypothesis that the specific architecture of the 8p amplicon determines FGFR dependency, the amplicon centered on *FGFR1* and *NSD3* in the sensitive cell lines, while in the resistant cell lines the amplification was neither focal nor centered on *FGFR1* (**Fig. 2b**). Of note, the inventors found one destructive *NSD3* breakpoint in the most sensitive cell line H1581, rearranging exon 15 of *NSD3* to an out of frame fusion with exon 10 of *ANK1.*

Furthermore, the inventors analysed patient-derived 8p12-8p11-amplified squamous cell lung cancer xenotransplant models. In total, the inventors performed *FGFR1* fluorescence in situ hybridization (FISH) on 35 patient derived xenograft (PDX) models from EpoBerlin, 39 PDX models from Crown Bioscience Inc., 3 samples of Moro Massimo as previously described. In addition, the inventors established 8 PDX models by implanting subcutaneously 2-3mm³ tumor pieces from biopsies (transported in DMEM media) together with 5-10µl of Matrigel (Corning) in NSG mice. The inventors identified eight *FGFR1* amplified models and performed CAGE sequencing. CAGE sequenced samples confirming *FGFR1* amplification were daily oral treated with BGJ398 (Tocris Bioscience, 20mg/kg, dissolved in 33% PEG300, 5% Glucose, prepare stock freshly every week and store at 4°C) or vehicle (33% PEG300, 5% Glucose) with at least three animals per group. Three of the 8 patient derived xenotransplant (PDX) models were sensitive to FGFR inhibition (**Fig. 2c**). Analysis of chromosomal gene copy number extracted from genomic sequencing data again revealed a pattern of amplification that was centered on *NSD3* and *FGFR1* in the sensitive PDX models (**Fig. 2d**). Of note, the inventors observed in one sensitive model a deleterious head-to-head *NSD3* rearrangement, deleting exons 9 to 23, but not in any of the insensitive models. By contrast, in insensitive PDX models 8p amplification was not centered on *FGFR1* nor focal within the 8p11-8p12 locus and the inventors found a destructive head-to-head rearrangement affecting the *FGFR1* gene, deleting exons 6 to 18 in one insensitive PDX, but not in any of the sensitive models.

Finally, the inventors analyzed biopsy specimens obtained from 10 patients with *FGFR1-*amplified squamous cell lung cancer. The samples were formalin fixed biopsies, taken before treatment. Eight of these patients had been treated with the FGFR-Inhibitor, BGJ398, as part of a phase I clinical trial (**Fig. 2e**) and one patient had responded to off-label use of pazopanib, a multi-kinase inhibitor that also inhibits FGFR. Of these 10 patients, 4 had experienced a partial response lasting for 9-17 months (defined as tumor shrinkage of at least 30% of the maximal tumor diameter) and 6 patients had progressive disease under treatment with an FGFR inhibitor. Given the low amount of available DNA from patient samples, the inventors developed a hybrid capture based sequencing assay tailored to cover much of the genomic 8p11-8p12 locus, as well as additional 226 genes. The inventors sequenced the 10 clinical specimens with an average sequencing depth of 470x using this approach. The inventors found three different *PIK3CA* mutations (G118S, E545K and H1047R) in addition to non-focal gain of 8p in 3 of 5 tumor specimens obtained from patients who had not experienced a response to the FGFR-inhibitor, BGJ398. Generally, patients who had not responded to FGFR inhibition, exhibited amplification centered on ADAM-family genes (**Fig. 2e****, f**). By contrast, detailed analyses of chromosomal gene copy number revealed focal amplifications centered on *FGFR1*/ *NSD3* in patients with a measurable response to FGFR inhibition (**Fig. 2e****, f**). Furthermore, the inventors identified one deleterious head-to-head break within *NSD3* in the responding patient group (1 in 9 samples, deleting exons 6-23).

In a pooled analysis of patients treated with FGFR inhibitors, PDX models and cell lines, the inventors found that focal and *FGFR1*-centered amplification was induced by tail-to-tail breaks close to the *FGFR1* transcriptional start side. These breaks occurred in 7 out of 9 (78%) "responders" (i.e., tumors/ cell lines that were sensitive to FGFR inhibition) and 3 out of 12 (25%) non-responders (i.e., insensitive tumors/ cell lines, for 5 cell line samples only whole exome sequencing data were available). Thus, tail-to-tail breaks close to the transcription start site of *FGFR1* induce focal amplification and were associated with sensitivity to FGFR inhibition (*p* = 0.03, Fisher's exact test). Furthermore, the inventors found three destructive *NSD3* breaks in sensitive samples (33%), deleting exons 6 to 23, but not in any of the insensitive models.

In tumors obtained from two of the four sensitive patients, the inventors identified tail-to-tail rearrangements deleting the first exons of *FGFR1,* including the canonical ATG start codon (**Fig. 2g****-j**), thus suggesting a possibly deleterious genomic event. In the first patient, the rearrangement occurred between exons 2 and 3 (**Fig. 2h**). The break in the second patient occurred between exons 1 and 2 (**Fig. 2j**). The inventors therefore speculated that the remaining part of the ORF of *FGFR1* might be preserved by an alternative start codon downstream. In support of this notion, the inventors were able to obtain tissue of one of these two specimens, which stained positive for pFGFRi, thus indicating intact and activated FGFR1 signaling (**Fig**. **2g****, right panel**).

### Example 3: Identification of intra-chromosomal tail-to-tail break from the non-coding region between FGFR1/NSD3 to FGFR1

As an additional support for the inventors' hypothesis, the inventors identified a similar break in the aforementioned tumor with *NSD3*/*FGFR1*-centered amplification that lacked a deleterious break in *NSD3* (**Fig. 3a**). This tumor exhibited a highly covered tail-to-tail break close to the transcription start side of *FGFR1,* indicating an early event by break amplification, and an intra-chromosomal tail-to-tail break within *FGFR₁* to a non-coding region. The break deleted exons 1 to 7 of *FGFR₁* including the canonical ATG start codon (**Fig. 3a**, **b**). However, this sample revealed a 50% higher transcription of *FGFR₁* (18 Fragments Per Kilobase Million) compared to *NSD3*_{long} (12 Fragments Per Kilobase Million) and revealed transcription across the breakpoint. Furthermore, the inventors confirmed transcription of the rearranged gene by reverse transcriptase PCR using primers covering the break point followed by sequencing (**Fig. 3c**). Finally, the inventors identified an additional *FGFR1*-amplified adenosquamous carcinoma with a tail-to-tail break within *FGFR₁* (**Fig. 3d**). This break led to deletion of exons 1 to 8 of *FGFR₁* (**Fig 3e**). Again, supporting the inventors' hypothesis of intact *FGFR₁* gene expression in the context of N-terminal deletions, the inventors found active transcription across the breakpoint (**Fig. 3f**). In this tumor, too, the inventors observed positive staining for pFGFRi in immunochemistry. Thus, all 4 tail-to-tail breaks within *FGFR₁* led to genomic deletions of exons of the 5' part of the gene (up to exon 8) accompanied by expression of N-terminally truncated versions of *FGFR₁* lacking the ectodomain, as well as amplification of the region encoding the transmembrane and kinase domains (exons 9-18).

The N-terminal part of FGFR₁ (comprising the ectodomain) is responsible for ligand specificity, as well as receptor autoinhibition. Furthermore, exon 9 has an in-frame non-canonical ATG start codon and deletion of the whole FGFR₁ ectodomain leads to ligand independent dimerization. The inventors therefore hypothesized that N-terminal deletion of the ectodomain of FGFR₁ by intragenic tail-to-tail rearrangements might generate oncogenic versions of the kinase that cause FGFR₁ dependency and thus, sensitivity to FGFR inhibition.

The inventors used immunoblotting to analyze whether N-terminal deletion of the ectodomain of FGFR₁ by intragenic tail-to-tail rearrangements generates oncogenic versions of the kinase that cause FGFR₁ dependency. Briefly, cells were washed with cold PBS and lysed in RIPA Lysis Buffer supplemented with protease (Roche) and phosphatase inhibitor (Calbiochem) cocktails. After 20 minutes of incubation on ice, lysates were centrifuged at 18,000 g for 25 minutes. Protein concentration in supernatants was measured using BCA Protein Assay (ThermoScientific). Equivalent amounts of protein (30-60µg) were denatured and separated on 4-12% SDS-PAGE gels and after blotting on nitrocellulose membranes (Amersham Hybond-C Extra). The following antibodies were used for immunoblotting: β-actin (MP Bioscience); phospho-FGFR (Tyr653, Tyr654), phospho-AKT (Ser473), AKT, phospho-ERK, and total ERK, total FGFR₁ (Abcam), and conjugated antibodies to rabbit and mouse (Millipore).

The inventors further observed that ectopic expression of ΔEC-FGFR1 in murine Ba/F₃ cells led to IL₃-independent cell growth. By contrast, overexpression of wild-type (wt) FGFR₁ was not sufficient to induce IL-3 independence. Furthermore Ba/F₃ cells expressing ΔEC-FGFR1 were highly sensitive to FGFR inhibition by the FGFR inhibitors, BGJ398 and AZD4547 (**Fig. 3g**, **h, i**).

In summery, the inventors discovered that 8p11-8p12 amplifications in squamous cell lung cancer are typically caused by Breakage Fusion Bridges. The mechanism of amplification induces head-to-head followed by tail-to-tail intra-chromosomal breaks within the 8p arm. Based on this mechanism, the inventors were able to reconstruct the observed 8p11-p12 copy number profile, taking all genomic breaks within the 8p-arm into account. Furthermore, tail-to-tail breaks close to the transcription start side of *FGFR₁* led to focal and high-amplitude amplification, centered on *FGFR1*/*NSD3* and were associated with sensitivity to FGFR inhibition. The corresponding head-to-head break was frequently located within *NSD₃* (*WHSC1L1*)*,* which led to deletion of exons 6 to 23, thereby deleting the catalytic SET domain of NSD3 (exons 19-20). Taking all 52 samples together, 8 out of 9 *NSD₃* breaks (89%) were detected in 18 samples with focal *FGFR₁* amplification driving expression of non-oncogenic NSD3ₛₕₒᵣₜ (exons 1-10). By contrast, in 4 *FGFR1*-amplified samples (8% of *FGFR₁* amplified samples used in this study) a tail-to-tail break occurred within the open reading frame (ORF) of *FGFR₁* deleting various portions of the gene ranging from exons 1-8. These breaks induced expression of a short and oncogenic version of FGFR₁ lacking the ectodomain using a non-canonical in frame ATG start codon in exon 9. Mechanistically, ligand-independent dimerization by FGFR₁ variants lacking the ectodomain has been previously reported. However, somatic genomic alterations that cause such variants have not been described to date.

To proof expression over the breakpoint, validate the tail-to-tail rearrangement, as well as the ΔEC-FGFR1 variant, the inventors generated cDNA from 1µg total RNA of the sample S00674 and its corresponding normal (provided from Elisabeth Brambilla, Grenoble). cDNA was generated using the SuperScripIII kit (Invirtrogen) following manufacturer's instructions followed by PCR using the break spanning primers 191_F1_S00674/201_R9_S00674 and 193_F2_S00674/ 202_R10_S00674. A head-to-head rearrangement was validated in this sample by a nested PCR using only one primer per PCR run (based on the palindromic nature of this break). In the first run the inventors used the primer 261_6_R1. In the second run (4µl Template from first PCR) the inventors used the primer 262_6_R2. The expected band size was 794 base pairs (based on the whole genome sequencing data). cDNA of H1581 cells (100ng) was used to amplify *FGFR1* by attB-overhang primers and flip it into pDONR.221 using the BP-clonase (Invitrogen). Bacterial transformation of the competent E. coli strain DH5α (Invitrogen) was carried out according to the manufacturer's instructions. Single clones were sequenced from mini-preparation of plasmid DNA using the NucleoSpin Mini Kit (Machery Nagel). For midi-preparation of plasmid DNA the inventors used the NucleoBond Xtra Midi EF Kit (Machery Nagel). The different *FGFR1* variants were generated by side using Gibson Assembly and the primers 234_F_ΔEC-21-FGFR1, 235_F_AEC-30-FGFR1, 236_F_ΔEC-85-FGFR1, 237_F_ΔEC-144-FGFR1, and 211_R_FGFR1_GA.

Conclusively, the inventors' findings suggest that intra-chromosomal tail-to-tail breaks in *FGFR1*

## Claims

1. **A method for identifying a subject suffering from cancer as a responder or non-responder to fibroblast growth factor receptor 1 (FGFR1) inhibitor therapy,** the method comprising the steps of
**a)** Providing from the subject a biological sample comprising genetic material associated with the cancer,
**b)** Determining in the genetic material the presence or absence of a genetic alteration of the *FGFR1* gene, wherein the genetic alteration is a copy number variation caused by a chromosomal break, such as a Breakage-Fusion-Bridge-like (BFB-like) mechanism, wherein the break is located within, or within close proximity of, the ORF of the *FGFR1* gene;
wherein the presence of the genetic alteration in the *FGFR1* gene in the biological sample of the subject indicates that the subject is a responder to FGFRi-inhibitor therapy.

2. The method according to claim 1, wherein the cancer is a solid cancer, preferably a cancer **characterized by** the expression of FGFR1, such as breast cancer, a gastric cancer, bladder cancer or lung cancer, and most preferably is Non-small cell lung cancer (NSCLC), such as squamous cell lung cancer (SQLC).

3. The method according to claim 1 or 2, wherein the subject is a human, preferably a human patient diagnosed with NSCLC, such as SQLC.

4. The method according to any one of claims 1 to 3, wherein the biological sample comprises tumour cells of the subject, preferably wherein the biological sample is a tumour biopsy, or a sample of a resected primary tumour.

5. The method according to any one of claims 1 to 4, wherein the FGFR1 inhibitor is a selective or unselective FGFR1 inhibitor, such as an inhibitor selected from the group consisting of NVP-BGJ398, AZD4547, TKI258, JNJ42756493, pemigatinib, lucitanib, Rogaratinib, Debio 1347, FIIN2, LY2874455, PRN1371 and ponatinib.

6. The method according to any one of claims 1 to 5, wherein the genetic alteration is
**a)** a genetic modification of the *FGFR1* locus resulting in a truncation of at least a part of an extracellular domain of FGFR1, and /or
**b)** a genetic copy number gain of a FGFR1 gene, or a modified variant thereof; and/or
**c)** a consecutive palindromic arrangement of the *FGFR1 gene,* or a partial *FGFR1* gene; and/or
**d)** a genetic modification of the FGFR1 locus resulting in the translation initiation of a non-canonical ATG start-codon.

7. The method according to any one of claims 1 to 6, wherein the genetic alteration is an alteration of the *FGFR1* gene resulting in the expression of an ectodomain-deleted variant of FGFR1, preferably wherein the variant of FGFR1 is translated from a non-canonical ATG in exon 1 to exon 9 of *FGFR1.*

8. **An inhibitor of FGFR1 for use in the treatment of cancer in a patient,** wherein the cancer is **characterized by** a genome which comprises a genetic alteration in the *FGFR1* gene comprising a consecutive palindromic copy number variation of the *FGFR1* gene, or of a fragment of the *FGFR1* gene, optionally wherein the cancer is a solid cancer, preferably a cancer **characterized by** the expression of FGFR1, such as breast cancer, a gastric cancer, bladder cancer or lung cancer, and most preferably is Non-small cell lung cancer (NSCLC), such as squamous cell lung cancer (SQLC).

9. The inhibitor of FGFR1 for use according to claim 8, wherein the inhibitor of FGFR1 is a small molecular inhibitor, an antigen binding protein, such as an antibody, or any antibody derivative, preferably which specifically binds to and inhibits FGFR, preferably FGFR1, optionally wherein the inhibitor of FGFR1 is a selective or unselective FGFR1 inhibitor, such as an inhibitor selected from the group consisting of NVP-BGJ398, AZD4547, TKI258, JNJ42756493, pemigatinib, lucitanib, Rogaratinib, Debio 1347, FIIN2, LY2874455, PRN1371 and ponatinib.

10. The inhibitor of FGFR1 for use according to claim 8 or 9, wherein the genetic alteration is a focal amplification of the *FGFR1* gene, or of a fragment of the *FGFR1* gene, optionally wherein the fragment of the *FGFR1* gene is an expressible sequence and results in a FGFR1 protein variant, preferably an ectodomain-deleted FGFR1 protein variant.

11. The inhibitor of FGFR1 for use according to any one of claims 8 to 10, wherein the treatment comprises a preliminary step of identifying the patient as a responder to an FGFR1 inhibitor therapy, wherein the preliminary step comprises a method according to any one of claims 1 to 7.

12. **A method for the diagnosis, prognosis, stratification and/or monitoring of a therapy, of cancer in a subject,** comprising the steps of:
**a)** Providing a biological sample from the subject, and
**b)** Determining in the biological sample the presence or absence of a chromosomal break, such as a Breakage-Fusion-Bridge-like (BFB-like) mechanism, for example a tail-to-tail BFB-like mechanism, wherein the break is located within, or within close proximity of, the ORF of the *FGFR1* gene, an alteration of the mRNA-transcript of the *FGFR1* gene, an alteration of the FGFR1 protein, an altered level of an mRNA-transcript of the *FGFR1* gene, and/or an altered level of the FGFR1 protein, preferably wherein the extracellular domain of FGFR1 is deleted,
wherein the presence of the chromosomal break, the alteration of the mRNA-transcript of the *FGFR1* gene, the alteration of the FGFR1 protein, a differential level of an mRNA-transcript of the *FGFR1* gene, and/or a differential level of the FGFR1 protein as determined in step **(b)** compared to a healthy control or reference value is indicative for the presence of cancer in the subject.

13. The method according to claim 12, wherein the chromosomal break induces:
(i) a genetic modification of the *FGFR1* locus resulting in a truncation of at least a part of an extracellular domain of FGFR1, and/or
(ii) a focal amplification of *FGFR1,* or of a fragment of the *FGFR1* gene; and/or
(iii) a consecutive palindromic arrangement of the *FGFR1 gene,* or a partial *FGFR1* gene; and/or
(iv) a genetic modification of the FGFR1 locus resulting in the translation initiation of a non-canonical ATG start-codon.

14. The method according to claim 12 or 13, wherein the genetic modification, and/or the alteration of the mRNA-transcript of the *FGFR1* gene comprises a nucleic acid sequence according to any one of SEQ ID Nos: 2, 3, 10, 11, 15, 16, 20, 21, 25, 28, 31, 34, or 37, and/or wherein the altered FGFR1 protein comprises an amino acid sequence according to any one of SEQ ID Nos: 9, 14, 19, 24, 26, 29, 32, or 35.

15. The method according to any one of claims 12 to 14, wherein the cancer is a solid cancer, preferably a cancer **characterized by** the expression of FGFR1, such as breast cancer, a gastric cancer, bladder cancer or lung cancer, and most preferably is Non-small cell lung cancer (NSCLC), such as squamous cell lung cancer (SQLC).
